# EUROPEAN PATENT SPECIFICATION

(11) **EP 4 333 854 B1**
(45) Date of publication and mention of the grant of the patent: **15.07.2026**
(21) Application number: 22725908.2
(22) Date of filing: 03.05.2022
(51) Int. Cl.: A61K 31/538, A61P 31/04, C07D 401/04, C07D 413/14

(54) **ANTIBACTERIAL COMPOUND**
ANTIBAKTERIELLE VERBINDUNG
COMPOSÉ ANTIBACTÉRIEN

(30) Priority: 03.05.2021 IN 202111020227; 24.06.2021 EP 21181594; 05.04.2022 GB 202204981
(43) Date of publication of application: 13.03.2024
(73) Proprietor: Discuva Ltd., Milton Park Abingdon Oxfordshire OX14 4SB (GB)
(72) Inventor: KHAN, Mohammed Nawaz, Abingdon Oxfordshire OX14 4SB (GB); BREIDENSTEIN, Elena Bernadette Monika, Abingdon Oxfordshire OX14 4SB (GB); BYE, Alan, Horsham Sussex RH12 5TQ (GB)
(74) Representative: Gill Jennings & Every LLP
(86) International application number: PCT/EP2022/061873
(87) International publication number: WO 2022/233886

(56) References cited:
- WO-A1-2019/086890

## Description

### Field of the Invention

The present invention relates to an antibacterial compound as defined herein, to pharmaceutical compositions containing the compound, and to the use of the compound and pharmaceutical compositions containing the compound in the treatment of bacterial infection with, or disease caused or exacerbated by, Gram-negative bacteria of the order Enterobacterales, in particular those that have developed resistance to existing antibiotics.

### Background of the Invention

There is a need for new antibacterial compounds to counter the emergence of bacterial pathogens with resistance to existing antibacterial compounds. The increasing occurrence of bacterial resistance to existing antibiotics threatens to greatly enhance the burden that common infections place on society, with multidrug resistance becoming common amongst a number of bacterial pathogens. For example, antibiotic-resistant strains of the ESKAPE pathogens (*Enterococcus faecium, Staphylococcus aureus, Klebsiella pneumoniae, Acinetobacter baumannii, Pseudomonas aeruginosa* and *Enterobacter* species), such as carbapenem-resistant Enterobacterales (CRE), multi-drug resistant (MDR) *Acinetobacter,* MDR *Pseudomonas aeruginosa*, methicillin-resistant *Staphylococcus aureus* (MRSA) and vancomycin-resistant *Enterococcus* (VRE) have been included in a list of antibiotic-resistant microorganisms identified as posing an urgent or serious threat to human health. Other prominent antibiotic-resistant pathogens include the Gram-positive anaerobe *Clostridium difficile*, drug-resistant *Neisseria gonorrhoeae* and drug-resistant *tuberculosis*.

Specifically, the resistance of Gram-negative bacteria, in particular of the order Enterobacterales, to existing antibiotic agents such as carbapenem (β-lactam) antibiotics is increasing. Antibiotic-resistant Gram-negative Enterobacterales strains, such as carbapenemase-producing Enterobacterales e.g. *Escherichia coli* NDM-1 (New Delhi metallo-β-lactamase) and *Klebsiella pneumoniae*, are difficult to treat and are becoming increasingly widespread and virulent. Moreover, new emerging hyper-virulent, multidrug resistant and highly transmissible strains of carbapenem-resistant *Klebsiella pneumoniae* associated with fatal outbreaks have been identified, for example, ST11 carbapenem-resistant hypervirulent *Klebsiella pneumoniae* strains. Such strains are resistant to previously and currently recommended antibiotics and are now a global major public health concern.

Currently, Enterobacterales infections where carbapenem-resistant Enterobacterales (CRE) are suspected, are commonly treated with broad spectrum agents. Such treatment typically consists of a combination of a β-lactam antibiotic and a β-lacatamase inhibitor (BL/BLI), or the use of colistin, an antibiotic of last resort. The BL/BLI combinations, whilst effective in the short term, are predicted to eventually succumb to pre-existing resistance mechanisms of the bacteria. Furthermore, these broad spectrum agents are not active against all Ambler β-lactamase classes, having no activity against metallo-β-lactamases, which confer resistance to a broad range of β-lactam antibiotics, including carbapenem antibiotics.

There is therefore a need for new antibacterial compounds that can provide effective treatment in a reliable manner for an infection with, or disease caused or exacerbated by, Gram-negative bacteria of the order Enterobacterales which are resistant to known antibiotics such as carbapenems and other β-lactams, or involving multidrug resistant infection agents. There is additionally a need for the provision of antibiotic agents that can avoid or reduce the side-effects associated with known antibacterial compounds.

It is an object of the aspects of the present invention to provide a solution to the above mentioned or other problems.

WO 2019/086890 A1 discloses compounds that find application in the treatment of infection with, and diseases caused by, *Enterobacteriaceae*.

### Summary of the Invention

According to a first aspect of the present invention there is provided a compound (I): 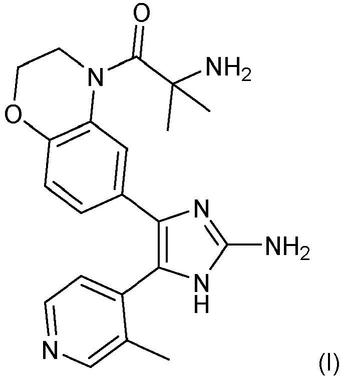 or a pharmaceutically acceptable salt, hydrate, or solvate thereof.

According to a second aspect of the present invention, there is provided a compound (I): 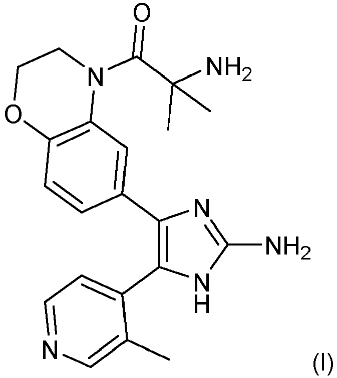 or a pharmaceutically acceptable salt, hydrate, or solvate thereof, for use in therapy or prophylaxis of an infection with, or disease caused or exacerbated by, Gram-negative bacteria of the order Enterobacterales and/or Gram-negative bacteria of the genus *Haemophilus.*

Also disclosed herein, there is a compound (I): 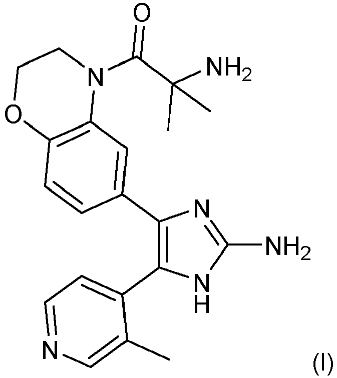 or a pharmaceutically acceptable salt, hydrate, or solvate thereof, for use in a method of treatment of an infection with, or disease caused or exacerbated by, Gram-negative bacteria of the order Enterobacterales and/or Gram-negative bacteria of the genus *Haemophilus.*

Also disclosed herein, there is a compound (I): 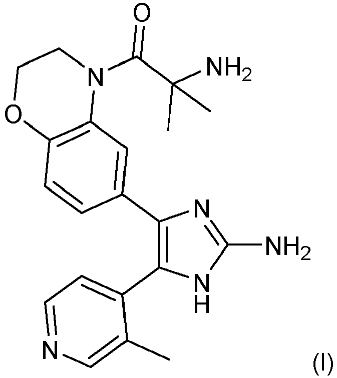 or a pharmaceutically acceptable salt, hydrate, or solvate thereof, for the manufacture of a medicament for use in the treatment of an infection with, or disease caused or exacerbated by, Gram-negative bacteria of the order Enterobacterales and/or Gram-negative bacteria of the genus *Haemophilus.*

Also disclosed herein, there is provided a method of treating an infection with, or disease caused or exacerbated by, Gram-negative bacteria of the order Enterobacterales and/or Gram-negative bacteria of the genus *Haemophilus,* in a subject in need thereof, comprising administering to said subject an effective amount of a compound (I): 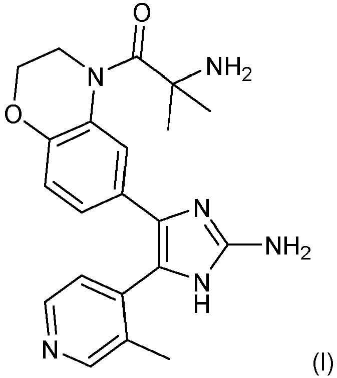 or a pharmaceutically acceptable salt, hydrate, or solvate thereof.

The compound (I) has bactericidal activity against Gram-negative bacteria of the order Enterobacterales and/or Gram-negative bacteria of the genus *Haemophilus,* and may be used in the treatment or prophylaxis of an infection with, or disease caused or exacerbated by, Gram-negative bacteria of the order Enterobacterales and/or Gram-negative bacteria of the genus *Haemophilus.*

According to a further aspect of the present invention, there is provided a compound (I): 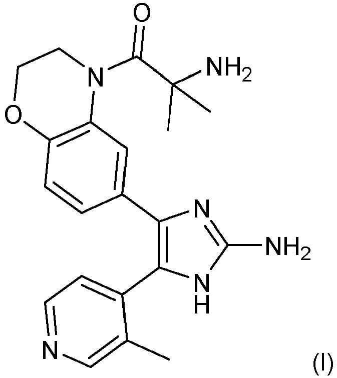 or a pharmaceutically acceptable salt, hydrate, or solvate thereof, for use in the treatment of infection with, or disease caused or exacerbated by, Gram-negative bacteria of the order Enterobacterales and/or Gram-negative bacteria of the genus *Haemophilus.*

According to a further aspect of the present invention, there is provided a method of *in vitro* inhibition of the growth of Gram-negative bacteria of the order Enterobacterales and/or Gram-negative bacteria of the genus *Haemophilus* using compound (I): 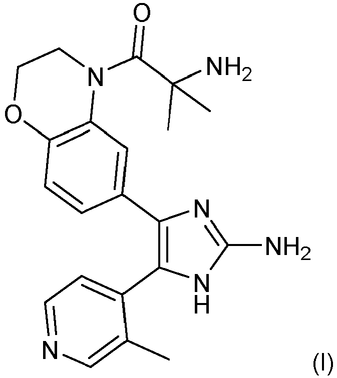 or a pharmaceutically acceptable salt, hydrate, or solvate thereof.

According to a further aspect of the present invention, there is provided a compound (I): 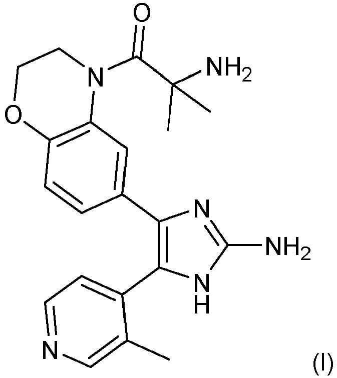 or a pharmaceutically acceptable salt, hydrate, or solvate thereof, formulated together with a pharmaceutically acceptable excipient or carrier.

According to a further aspect of the present invention, there is provided a pharmaceutical composition comprising compound (I): 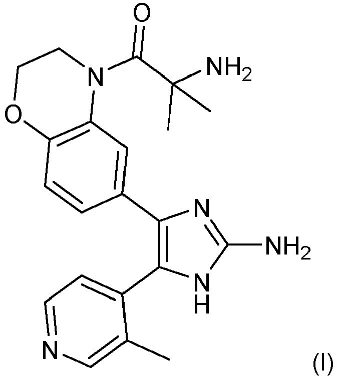 or a pharmaceutically acceptable salt, hydrate, or solvate thereof, and a pharmaceutically acceptable excipient or carrier.

According to a further aspect of the present invention, there is provided a pharmaceutical composition for use in the treatment of infection with, or disease caused or exacerbated by, Gram-negative bacteria of the order Enterobacterales and/or Gram-negative bacteria of the genus *Haemophilus,* the pharmaceutical composition comprising a compound (I), or a pharmaceutically acceptable salt, hydrate, or solvate thereof, and a pharmaceutically acceptable excipient or carrier, wherein compound (I) is:

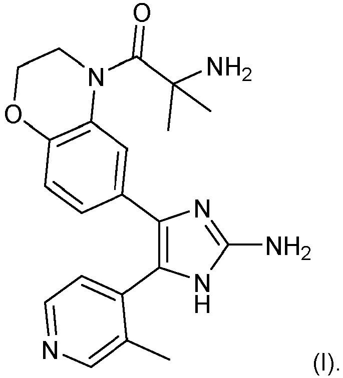

According to a further aspect of the present invention, there is provided a pharmaceutical composition for use in the treatment of infection with, or disease caused or exacerbated by, Gram-negative bacteria of the order Enterobacterales and/or Gram-negative bacteria of the genus *Haemophilus,* the pharmaceutical composition comprising a compound (I), or a pharmaceutically acceptable salt, hydrate, or solvate thereof, and a pharmaceutically acceptable excipient or carrier, wherein compound (I) is: 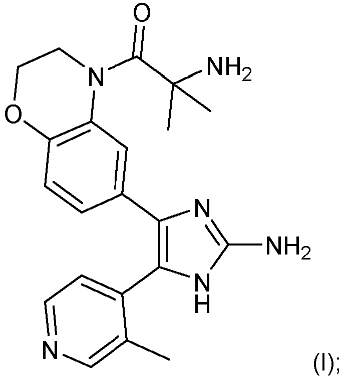 and wherein the pharmaceutical composition is administered by intravenous infusion, with the compound (I), or pharmaceutically acceptable salt, hydrate, or solvate thereof, at a dose of 50 to 6000 mg per day.

### Definitions

As used herein, the term "disease" or "bacterial disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from trauma, injury, surgery, radiological ablation, poisoning or nutritional deficiencies. The term refers to any disease that involves (e.g. is caused, exacerbated, associated with or characterized by the presence of) a bacterium residing and/or replicating in the body and/or cells of a subject. The term therefore includes diseases caused or exacerbated by bacterial toxins (which may also be referred to herein as "bacterial intoxication").

As used herein, the term "infection" or "bacterial infection" is used to define a condition in which a subject is infected with a bacterium. The infection may be symptomatic or asymptomatic. In the former case, the subject may be identified as infected on the basis of established diagnostic criteria. In the latter case, the subject may be identified as infected on the basis of various tests, including for example biochemical tests, serological tests, microbiological culture and/or microscopy. Thus, the invention typically finds application in the treatment of subjects in which a bacterial infection has been diagnosed or detected.

As used herein, the term "treatment" or "treating" refers to an intervention (e.g. the administration of an agent to a subject) which cures, ameliorates or lessens the symptoms of a disease or removes (or lessens the impact of) its cause(s) (for example, the causative bacterium). In this case, the term is used synonymously with the term "therapy". Thus, the treatment of infection according to the invention may be characterized by the (direct or indirect) bactericidal action of the compounds of the invention. Thus, the compounds of the invention find application in methods of killing, or preventing the growth of, bacterial cells.

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

The term "subject" (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Preferably, the subject is a human.

The terms "Gram-negative bacterium" and "Gram-positive bacterium" are terms of art defining two distinct classes of bacteria on the basis of certain cell wall staining characteristics.

As used herein, an "effective amount" of a compound or agent is an amount to achieve a desired pharmacologic effect or therapeutic improvement without undue adverse side effects. A "therapeutically effective amount," as used herein, refers to a sufficient amount of an agent or a compound being administered which will relieve to some extent one or more of the symptoms of an infection or disease. The result can be reduction and/or alleviation of the signs, symptoms, or causes of the infection or disease or any other desired alteration of a biological system. A therapeutic result need not be a complete cure. The term "therapeutically effective amount" includes, for example, a prophylactically effective amount. It is understood that "an effective amount" or "a therapeutically effective amount" can vary from subject to subject, depending on the age, weight, general condition of the individual, mode of administration, the condition being treated, the severity of the condition being treated and other factors.

As used herein, a "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The term "efficacious" includes advantageous effects such as additivity, synergism, reduced side effects, reduced toxicity, improved performance, reduced bacterial burden during infection, or activity.

The term "pharmaceutically acceptable salt" as applied to the compound (I) of the invention defines any organic or inorganic acid addition salt of the free base which are suitable for use in contact with the tissues of humans and animals without undue toxicity, irritation, allergic response and which are commensurate with a reasonable benefit/risk ratio. Suitable pharmaceutically acceptable salts are well known in the art. Examples are the salts formed from inorganic acids (for example hydrochloric, hydrobromic, sulphuric and phosphoric acids), organic carboxylic acids (for example acetic, propionic, glycolic, lactic, pyruvic, malonic, succinic, fumaric, malic, tartaric, citric, ascorbic, maleic, hydroxymaleic, dihydroxymaleic, benzoic, phenylacetic, 4-aminobenzoic, 4-hydroxybenzoic, anthranilic, cinnamic, salicylic, 2-phenoxybenzoic, 2-acetoxybenzoic and mandelic acid) and organic sulfonic acids (for example methanesulfonic acid and p-toluenesulfonic acid).

The term "solvate" as applied to the compound (I) of the invention means a physical association of compound (I) with one or more solvent molecules, whether organic or inorganic. This physical association can include hydrogen bonding. In certain instances the solvate will be capable of isolation, for example when one or more solvent molecules are incorporated in the crystal lattice of the crystalline solid. The solvate may comprise either a stoichiometric or nonstoichiometric amount of the solvent molecules. For example, a solvate with a nonstoichiometric amount of solvent molecules may result from partial loss of solvent from the solvate. "Solvate" encompasses both solution-phase and isolable solvates.

Exemplary solvates include hydrates, ethanolates, methanolates, isopropanolates and the like. Methods of solvation are generally known in the art.

The term "hydrate" as applied to compound (I) of the invention refers to compound (I) when it is associated with water in the molecular form, i.e., in which the H-OH bond is not split, and may be represented, for example, by the formula R·H₂O, where R is a compound of the invention. A given compound may form more than one hydrate including, for example, monohydrates (R·H₂O) or polyhydrates (R·nH₂O wherein n is an integer >1) including, for example, dihydrates (R·2H₂O), trihydrates (R·3H₂O), and the like, or hemihydrates, such as, for example, R·n/2H₂O, R·n/3H₂O, R·n/4H₂O and the like wherein n is an integer.

In its broadest aspect, the present invention contemplates all optical isomers of the compound (I), or pharmaceutically acceptable salts, hydrates, or solvates.

In cases where the stereochemical form of the compound (I), or pharmaceutically acceptable salt, hydrate, or solvate is important for pharmaceutical utility, the invention contemplates use of an isolated eutomer.

The term "resistant strains" as used herein, refers to strains of bacteria that have shown resistance or non-susceptibility to one or more known antibacterial agent, particularly those widely considered to be standard-of-care in the treatment of infections with such strains. A "non-susceptible strain" is one in which the MIC (minimum inhibitory concentration) of a given compound or class of compounds for that strain has shifted to a higher number than for corresponding susceptible strains. For example, it may refer to strains that are non-susceptible to β-lactam antibiotics, strains that are non-susceptible to one or more fluoroquinolones and/or strains that are non-susceptible to one or more other antibiotics (i.e. antibiotics other than β-lactams and fluoroquinolones). In some instances, the term "resistant" may refer to one strain in which the MIC of a given compound or class of compounds for that strain has shifted to a significantly higher number than for corresponding susceptible strains. A bacterial strain might be said to be resistant to a given antibiotic when it is inhibited *in vitro* by a concentration of this agent that is associated with a high likelihood of therapeutic failure.

The term "multidrug resistant" or "multidrug resistance" as used herein, refers to organisms, such as highly resistant Gram-negative bacteria (e.g. carbapenemase-producing *Klebsiella pneumoniae*), showing *in vitro* and/or *in vivo* resistance to more than one antimicrobial agent. Such organisms may be resistant to all of the currently available antimicrobial agents or remain susceptible only to older, potentially more toxic, antimicrobial agents.

The term "hypervirulent" as used herein, refers to organisms that are exceptionally virulent, generally as a result of the acquisition of a virulence plasmid. Such organisms are capable of producing severe illness. For completeness, "virulent" refers to organisms capable of producing extremely severe or harmful effects and illness.

The term "antibacterial treatment" as used herein is meant treatment of the infection or disease of a subject with an antibacterial agent other than compound (I), or a pharmaceutically acceptable salt, hydrate, or solvate thereof. Typically, in the context of the present invention, this is with known antibiotics or treatment methods, including carbapenem or other β-lactam antibiotics, other than compound (I), or a pharmaceutically acceptable salt, hydrate, or solvate thereof.

### Detailed Description of the Invention

The present invention provides the solution to a previously unmet clinical need with regard to the treatment of infection with, or disease caused or exacerbated by Gram-negative bacteria of the order Enterobacterales, particularly carbapenem-resistant Enterobacterales (CRE) and extended spectrum β-lactamase (ESBL) Enterobacterales. The present invention may also be utilised in the treatment of infection with, or disease caused or exacerbated by Gram-negative bacteria of the genus *Haemophilus.*

Any references to methods of treatment in this description are to be interpreted as references to compounds and pharmaceutical compositions of the present invention for use in those methods.

It has been surprisingly and advantageously found that compound (I) or a pharmaceutically acceptable salt, hydrate, or solvate thereof, enables the treatment of infection with, or disease caused or exacerbated by Gram-negative bacteria of the order Enterobacterales and/or Gram-negative bacteria of the genus *Haemophilus,* including Gram-negative bacteria of the order Enterobacterales and/or Gram-negative bacteria of the genus *Haemophilus* which are resistant to known β-lactam antibiotics and a major cause of mortality.

Compound (I), or a pharmaceutically acceptable salt, hydrate, or solvate thereof, may be particularly advantageous in the treatment of multi-site infection, i.e. infections with Gram-negative bacteria of the order Enterobacterales and/or Gram-negative bacteria of the genus *Haemophilus,* and occurring at different infection sites (intracellular sites in tissues and/or organs) of a subject (shown by the data provided in the Examples section of the present invention). Compound (I), or a pharmaceutically acceptable salt, hydrate, or solvate thereof, may further be advantageously utilised in the treatment of infection with, or diseases caused or exacerbated by Gram-negative bacteria of the order Enterobacterales and/or Gram-negative bacteria of the genus *Haemophilus,* where antibacterial treatment of the disease or infection in the subject requiring treatment has failed, or where the subject is allergic or otherwise contra-indicated to any agents used, or considered for use, in antibacterial treatment.

Compound (I), or a pharmaceutically acceptable salt, hydrate, or solvate thereof, appears to be rapidly bactericidal and is considered highly active against all currently tested clinical mechanisms of resistance, including all Ambler β-lactamase classes (including metallo-β-lactams). Compound (I), or a pharmaceutically acceptable salt, hydrate, or solvate thereof, does not appear to be subject to deactivation by the full range of carbapenem metabolising enzymes, demonstrating activity against all currently tested CREs independent of the carbapenemase or other resistance characteristics. In contrast to known BL/BLI combination treatments, compound (I), or a pharmaceutically acceptable salt, hydrate, or solvate thereof, has shown a low propensity for tested resistance development *in vitro.*

Furthermore, compound (I), or a pharmaceutically acceptable salt, hydrate, or solvate thereof, displays no cross resistance with currently tested antibiotic classes, and no observed contraindication. Compound (I), or a pharmaceutically acceptable salt, hydrate, or solvate thereof, also demonstrates both *in vitro* and *in vivo* safety in pharmacology studies.

Accordingly, compound (I), or a pharmaceutically acceptable salt, hydrate, or solvate thereof, may be used in the treatment of bacterial infections and diseases caused or exacerbated by Gram-negative bacteria of the order Enterobacterales and/or Gram-negative bacteria of the genus *Haemophilus,* in particular, those bacteria resistant to known antibiotics, such as carbapenem-resistant Enterobacterales (CRE) and extended spectrum β-lactamase (ESBL) Enterobacterales. The compound (I), or a pharmaceutically acceptable salt, hydrate, or solvate thereof, has bactericidal activity against Gram-negative bacteria of the order Enterobacterales and/or Gram-negative bacteria of the genus *Haemophilus,* in particular, those bacteria resistant to known antibiotics, such as carbapenem-resistant Enterobacterales (CRE) and extended spectrum β-lactamase (ESBL) Enterobacterales.

Compound (I), or a pharmaceutically acceptable salt, hydrate, or solvate thereof, may target one or more Gram-negative bacteria of the order Enterobacterales. Compound (I), or a pharmaceutically acceptable salt, hydrate, or solvate thereof, may target one or more Gram-negative bacteria of families and genera of the Enterobacterales order of Gram-negative bacteria. The Enterobacterales order encompasses the *Enterobacteriaceae*, *Budviciaceae*, *Erwiniaceae*, *Hafniaceae*, *Morganellaceae*, *Pectobacteriaceae* and *Yersiniaceae* families, and all genera thereof. Compound (I), or a pharmaceutically acceptable salt, hydrate, or solvate thereof, may target one or more Gram-negative bacteria of the following genera of the Enterobacterales order: *Arsenophonus, Atlantibacter, Biostraticola, Brenneria, Buchnera, Budvicia, Buttiauxella, Cedecea, Chania, Citrobacter, Cosenzaea, Cronobacter, Dickeya, Edwardsiella, Enterobacillus, Enterobacter, Erwinia, Escherichia, Ewingella, Franconibacter, Gibbsiella, Hafnia, Izhakiella, Kosakonia, Klebsiella, Kluyvera, Leclercia, Lelliottia, Leminorella, Levinea, Lonsdalea, Mangrovibacter, Moellerella, Morganella, Obesumbacterium, Pantoea, Pectobacterium, Phaseolibacter, Photorhabdus, Plesiomonas, Pluralibacter, Pragia, Proteus, Providencia, Pseudocitrobacter, Rahnella, Raoultella, Rosenbergiella, Rouxiella, Saccharobacter, Salmonella, Samsonia, Serratia, Shigella, Shimwellia, Siccibacter, Sodalis, Tatumella, Thorsellia, Trabulsiella, Wigglesworthia, Xenorhabdus, Yersinia* and *Yokenella*.

Compound (I), or a pharmaceutically acceptable salt, hydrate, or solvate thereof, may target one or more Gram-negative bacteria of the genus *Haemophilus.* Gram-negative bacteria of the genus *Haemophilus* belong to the family *Pasteurellaceae*, and the order *Pasteurellales*.

Preferably, compound (I), or a pharmaceutically acceptable salt, hydrate, or solvate thereof, targets one or more Gram-negative bacteria of the order Enterobacterales. Preferably, compound (I), or a pharmaceutically acceptable salt, hydrate, or solvate thereof is used in the treatment of infection with, or disease caused or exacerbated by, Gram-negative bacteria of the order Enterobacterales.

Compound (I), or a pharmaceutically acceptable salt, hydrate, or solvate thereof, will typically have the same or similar activity against all Gram-negative bacteria of the order Enterobacterales. This is as a result of a sufficiently high sequence homology between the Gram-negative bacteria of the order Enterobacterales. Compound (I), or pharmaceutically acceptable salt, hydrate, or solvate thereof will therefore interact in a similar way with all of the Gram-negative bacteria of the order Enterobacterales. This is particularly the case for the selected Enterobacterales genera detailed below. Preferably, compound (I), or a pharmaceutically acceptable salt, hydrate, or solvate thereof, targets one or more Gram-negative bacteria from the Enterobacterales genera of Cedecea, *Citrobacter, Erwinia, Escherichia, Enterobacter, Klebsiella, Kluyvera, Plesiomonas, Proteus, Providencia, Raoultella, Salmonella, Serratia, Shigella,* and *Yersinia*. More preferably, compound (I), or a pharmaceutically acceptable salt, hydrate, or solvate thereof, targets one or more Gram-negative bacteria from the Enterobacterales genera of *Erwinia*, *Escherichia*, *Enterobacter*, *Klebsiella*, *Proteus*, *Salmonella*, *Serratia*, *Shigella*, and *Yersinia*. The *Escherichia* genus includes *Escherichia coli* such as extraintestinal pathogenic *Escherichia Coli* (ExPEC) strains and carbapenem-resistant *Escherichia Coli* strains, e.g. *Escherichia Coli* of sequence type ST131 and *Escherichia Coli* ATCC BAA-2469 (NDM-1 strain: American Type Culture Collection). The *Enterobacter* genus includes *Enterobacter spp..* The *Klebsiella* genus includes *Klebsiella pneumoniae* such as carbapenem-resistant *Klebsiella pneumonia* strains, e.g. *Klebsiella pneumonia* of sequence type ST258 and *Klebsiella pneumonia* ATCC 43816 (American Type Culture Collection). Preferably, compound (I), or a pharmaceutically acceptable salt, hydrate, or solvate thereof, targets one or more Gram-negative bacteria from the Enterobacterales genera of *Escherichia*, *Enterobacter,* and *Klebsiella,* more preferably *Escherichia* and *Klebsiella.* Preferably, compound (I), or a pharmaceutically acceptable salt, hydrate, or solvate thereof, targets *Enterobacter spp*., *Escherichia coli* and *Klebsiella pneumonia,* more preferably *Escherichia coli* and *Klebsiella pneumonia.*

The one or more Gram-negative bacteria targeted by compound (I), or a pharmaceutically acceptable salt, hydrate, or solvate thereof, are typically multidrug resistant, including carbapenem- or β-lactam resistant and display resistance to these known antibiotics and associated antibacterial treatments. Such bacteria include, for example carbapenem-resistant *Escherichia Coli* ATCC BAA-2469, ExPEC *Escherichia Coli* ST131, *Klebsiella pneumonia* ST258 and *Klebsiella pneumonia* ATCC 42816.

The National Collection of Type Cultures (NCTC) Strain Reference for ExPEC *Escherichia Coli* ST131 is NCTC 13441. Further detail regarding ExPEC *Escherichia Coli* ST131 is provided in: Pitout et al., 'Escherichia Coli ST131: a multidrug-resistant clone primed for global domination', F1000Research 2017, 6(F1000 Faculty Rev):195; Ciesielczuk et al., 'Trends in ExPEC serogroups in the UK and their significance', Eur J Clin Microbial Infect Dis (2016) 35:1661-1666; Day et al., 'Extended-spectrum β-lactamase-producing Escherichia coli in human-derived and foodchain-derived samples from England, Wales, and Scotland: an epidemiological surveillance and typing study', Lancet Infect Dis 2019, vol. 19; and Day et al., 'Population structure of Escherichia coli causing bacteraemia in the UK and Ireland between 2001 and 2010', J Antimicrob Chemother 2016, 71, 2139-2142.

The National Collection of Type Cultures (NCTC) Strain Reference for *Klebsiella pneumonia* ST258 is NCTC 13438. Further detail regarding *Klebsiella pneumonia* ST258 is provided in: Chen et al., 'Carbapenemase-producing Klebsiella pneumonia: molecular and genetic decoding', Trends Microbiol., December 2014, 22(12), 686-696.

Compound (I), or a pharmaceutically acceptable salt, hydrate, or solvate thereof, may be used to treat infections with Gram-negative bacteria of the order Enterobacterales and/or Gram-negative bacteria of the genus *Haemophilus,* which are in the form of a biofilm.

The disease or infection caused or exacerbated by Gram-negative bacteria of the order Enterobacterales and/or Gram-negative bacteria of the genus *Haemophilus* may involve intoxication with one or more bacterial toxins, including for example endotoxins, exotoxins and/or toxic enzymes. Thus, the compound (I), or a pharmaceutically acceptable salt, hydrate, or solvate thereof, finds application in the treatment of Enterobacterales and/or *Haemophilus* intoxication. In such instances, the treatment of intoxication with bacterial endotoxins, exotoxins and/or toxic enzymes, for example with endotoxins, exotoxins and/or toxic enzymes produced by Gram-negative bacteria of the order Enterobacterales and/or Gram-negative bacteria of the genus *Haemophilus,* is preferred.

Compound (I), or a pharmaceutically acceptable salt, hydrate, or solvate thereof, may be used, as described in the various aspects herein, in the treatment of the human body, i.e. the subject to be treated is a human.

Compound (I), or a pharmaceutically acceptable salt, hydrate, or solvate thereof, may also be used in the treatment of the animal body, i.e. the subject to be treated is an animal. In particular, to treat commercial animals such as livestock. Alternatively, compound (I), or a pharmaceutically acceptable salt, hydrate, or solvate thereof, may be used to treat companion animals such as cats, dogs, etc. It will be appreciated that treatment of the animal body will be carried out in a similar manner for all subjects, i.e. both humans and animals.

Preferably, compound (I), or a pharmaceutically acceptable salt, hydrate, or solvate thereof, is used in the treatment of the human body, i.e. the subject to be treated is a human.

Compound (I), or a pharmaceutically acceptable salt, hydrate, or solvate thereof, may be used in the treatment of infections with, or diseases caused or exacerbated by Gram-negative bacteria of the order Enterobacterales and/or the Gram-negative bacteria of the genus *Haemophilus.* Specifically, compound (I), or a pharmaceutically acceptable salt, hydrate, or solvate thereof, may be used in the treatment of bacteraemia or bloodstream infections with Gram-negative bacteria of the order Enterobacterales and/or Gram-negative bacteria of the genus *Haemophilus,* respiratory infections with Gram-negative bacteria of the order Enterobacterales and/or Gram-negative bacteria of the genus *Haemophilus,* urinary tract infections (UTIs) with Gram-negative bacteria of the order Enterobacterales and/or Gram-negative bacteria of the genus *Haemophilus* (typically complicated UTIs), pyelonephritis (a kidney infection) with Gram-negative bacteria of the order Enterobacterales and/or Gram-negative bacteria of the genus *Haemophilus,* and intra-abdominal infections with Gram-negative bacteria of the order Enterobacterales and/or Gram-negative bacteria of the genus *Haemophilus.* In particular, compound (I), or a pharmaceutically acceptable salt, hydrate, or solvate thereof, may be used in the treatment of bacteraemia or bloodstream infections with Gram-negative bacteria of the order Enterobacterales and/or Gram-negative bacteria of the genus *Haemophilus,* respiratory infections with Gram-negative bacteria of the order Enterobacterales and/or Gram-negative bacteria of the genus *Haemophilus,* urinary tract infections (UTIs) with Gram-negative bacteria of the order Enterobacterales and/or Gram-negative bacteria of the genus *Haemophilus* (typically complicated UTIs), pyelonephritis (a kidney infection) with Gram-negative bacteria of the order Enterobacterales and/or Gram-negative bacteria of the genus *Haemophilus,* and intra-abdominal infections with Gram-negative bacteria of the order Enterobacterales and/or Gram-negative bacteria of the genus *Haemophilus,* where antibacterial treatment of the infection has failed. It is further noted that compound (I), or a pharmaceutically acceptable salt, hydrate, solvate, complex, or protected form thereof, may be used in the treatment of bacteraemia or bloodstream infections with Gram-negative bacteria of the order Enterobacterales and/or Gram-negative bacteria of the genus *Haemophilus,* respiratory infections with Gram-negative bacteria of the order Enterobacterales and/or Gram-negative bacteria of the genus *Haemophilus,* urinary tract infections (UTIs) with Gram-negative bacteria of the order Enterobacterales and/or Gram-negative bacteria of the genus *Haemophilus* (typically complicated UTIs), pyelonephritis (a kidney infection) with Gram-negative bacteria of the order Enterobacterales and/or Gram-negative bacteria of the genus *Haemophilus,* and intra-abdominal infections with Gram-negative bacteria of the order Enterobacterales and/or Gram-negative bacteria of the genus *Haemophilus,* where the subject requiring treatment is allergic, or otherwise contra-indicated, to any agent used in antibacterial treatment.

Bacteraemia or bloodstream infections with Gram-negative bacteria of the order Enterobacterales and/or Gram-negative bacteria of the genus *Haemophilus* can cause conditions such as sepsis (also known as septicaemia).

Respiratory infections with Gram-negative bacteria of the order Enterobacterales and/or Gram-negative bacteria of the genus *Haemophilus* include those of the respiratory tract or lungs, including pneumonia. In the context of the present invention, pneumonia typically refers to hospital-acquired pneumonia and healthcare pneumonia including ventilator-associated pneumonia, or pneumonia that has resulted in the subject being hospitalised.

Urinary tract infections (UTIs) with Gram-negative bacteria of the order Enterobacterales and/or Gram-negative bacteria of the genus *Haemophilus* include uncomplicated and complicated UTIs, affecting the bladder (cystitis), urethra (urethritis) or kidneys (kidney infection).

Intra-abdominal infections (IAIs) with Gram-negative bacteria of the order Enterobacterales and/or Gram-negative bacteria of the genus *Haemophilus* can cause conditions such as peritonitis, diverticulitis, cholecystitis, cholangitis and pancreatitis.

Preferably, compound (I), or a pharmaceutically acceptable salt, hydrate, solvate, complex, or protected form thereof, may be used in the treatment of bacteraemia or bloodstream infections with Gram-negative bacteria of the order Enterobacterales and/or Gram-negative bacteria of the genus *Haemophilus,* respiratory infections with Gram-negative bacteria of the order Enterobacterales and/or Gram-negative bacteria of the genus *Haemophilus,* and urinary tract infections (UTIs) with Gram-negative bacteria of the order Enterobacterales and/or Gram-negative bacteria of the genus *Haemophilus* (typically complicated UTIs), and more preferably may be used in the treatment of respiratory infections with Gram-negative bacteria of the order Enterobacterales and/or Gram-negative bacteria of the genus *Haemophilus,* and urinary tract infections (UTIs) with Gram-negative bacteria of the order Enterobacterales and/or Gram-negative bacteria of the genus *Haemophilus* (typically complicated UTIs).

Compound (I), or a pharmaceutically acceptable salt, hydrate, or solvate thereof, may be effective in the treatment of multi-site infections, i.e. infections with Gram-negative bacteria of the order Enterobacterales and/or Gram-negative bacteria of the genus *Haemophilus* and occurring within different infection sites (intracellular sites in tissues and/or organs) of a subject (shown by the data provided in the Examples section of the present invention). This may be, for example, in both the bloodstream to treat a bacteraemia or bloodstream infection caused or exacerbated by Gram-negative bacteria of the order Enterobacterales and/or Gram-negative bacteria of the genus *Haemophilus,* and in the respiratory organs to treat a respiratory infection caused or exacerbated by Gram-negative bacteria of the order Enterobacterales and/or Gram-negative bacteria of the genus *Haemophilus.* It will be appreciated that the infection at the multi-sites will typically have been caused or exacerbated by the same species of Gram-negative bacteria of the order Enterobacterales and/or Gram-negative bacteria of the genus *Haemophilus.* Infection with Gram-negative bacteria of the order Enterobacterales and/or Gram-negative bacteria of the genus *Haemophilus* can simultaneously or subsequently occur in different tissues and/or organs of a subject. Compound (I), or a pharmaceutically acceptable salt, hydrate, or solvate thereof, may be used in multi-site treatment of infections with, or disease caused or exacerbated by, Gram-negative bacteria of the order Enterobacterales and/or Gram-negative bacteria of the genus *Haemophilus.*

As indicated above, compound (I), or a pharmaceutically acceptable salt, hydrate, or solvate thereof, may be utilised following unsuccessful empirical antibacterial treatment with known antibiotics, particularly those regarded as standard-of-care for the treatment of the purported infection. Accordingly, treatment using compound (I), or pharmaceutically acceptable salts, hydrates, or solvates thereof, may be instigated following determination that antibiotic-resistant Gram-negative bacteria of the order Enterobacterales and/or Gram-negative bacteria of the genus *Haemophilus* are responsible for the infection or disease in a subject.

Following determination of the presence of antibiotic-resistant Gram-negative bacteria of the order Enterobacterales and/or Gram-negative bacteria of the genus *Haemophilus* such as carbapenem-resistant Enterobacterales (CRE) or extended spectrum β-lactamase (ESBL) Enterobacterales which are resistant to known β-lactam antibiotics, compound (I), or a pharmaceutically acceptable salt, hydrate, or solvate thereof, may be utilised to treat the infection in the subject with, or caused or exacerbated by, these resistant Enterobacterales and/or *Haemophilus* strains.

Methods of determining the presence of antibiotic-resistant Gram-negative bacteria of the order Enterobacterales and/or Gram-negative bacteria of the genus *Haemophilus,* such as carbapenem-resistant Enterobacterales or extended spectrum β-lactamase (ESBL) Enterobacterales, will be well known to a skilled person. Suitable methods include that disclosed in: Al-Zahrani, 'Routine detection of carbapenem-resistant gram-negative bacilli in clinical laboratories', Saudi Medical Journal, 2018 Sept., 861-872.

Compound (I), or a pharmaceutically acceptable salt, hydrate, or solvate thereof, may be used in any clinical practice or treatment. For example, compound (I), or a pharmaceutically acceptable salt, hydrate, or solvate thereof, may be used in hospital settings, for example, for severely ill hospitalised subjects. Alternatively or additionally, compound (I) may be used in out-patient therapies, such as out-patient parenteral antimicrobial therapy (OPAT), or by subjects in domestic settings. As noted above, treatment with compound (I), or a pharmaceutically acceptable salt, hydrate, or solvate thereof, can occur following unsuccessful antibacterial treatment. However, compound (I), or a pharmaceutically acceptable salt, hydrate, or solvate thereof may also be used as the first instance antibacterial treatment, for example, in a setting with a high rate of infection, for example endemic or unit outbreaks.

Compound (I), or a pharmaceutically acceptable salt, hydrate, or solvate thereof, may be used in the treatment of hospital-acquired infections (HAls) with Gram-negative bacteria of the order Enterobacterales and/or Gram-negative bacteria of the genus *Haemophilus,* for example introduction by a central line or catheter.

Compound (I), or a pharmaceutically acceptable salt, hydrate, or solvate thereof, may be formulated as a pharmaceutical composition, with a pharmaceutically acceptable carrier(s) or excipient(s). Suitable pharmaceutically acceptable carrier(s) and excipient(s) will depend on the mode of administration of the pharmaceutical composition, which are described in more detail below. Typically, compound (I), or a pharmaceutically acceptable salt, hydrate, or solvate thereof, is administered in a pharmaceutical composition, further comprising a pharmaceutically acceptable carrier(s) or excipient(s).

Pharmaceutical compositions as claimed herein may further comprise pharmaceutically acceptable components selected from, but not limited to, stabilizers, antioxidants, colorants, diluents and combinations thereof. The components of the pharmaceutical compositions are chosen such that side effects are minimized and the performance of compound (I), or a pharmaceutically acceptable salt, hydrate, or solvate thereof, is not compromised to such an extent that treatment is ineffective.

Pharmaceutical compositions according to the present invention may be administered to the subject in need by any suitable route, including but not limited to: enteral administration such as oral, rectal, gastric or duodenal administration; parenteral administration (such as injection or intravenously by infusion); vaginal administration; buccal or sub-lingual administration; topical administration or inhalation.

Parenteral administration includes subcutaneous, intravenous, intradermal, intramuscular and intraperitoneal administration, as well as infusion techniques, such as in the form of sterile injectable aqueous or emulsions as well as oleaginous suspensions. Such suspensions can be formulated according to known art using suitable dispersing or wetting agents and suspending agents. A sterile injectable preparation can be a sterile injectable solution or suspension in a non-toxic parenterally acceptable diluent or solvent, for example a solution in 1,3- butanediol. Among acceptable vehicles and solvents that can be employed are water, Ringer's solution and isotonic sodium chloride solution. In addition, sterile fixed oils are conventionally employed as a solvent or suspending medium. For this purpose, any bland fixed oil may be employed, including synthetic mono- or diglycerides. In addition, omega-3 polyunsaturated fatty acids can find use in preparation of injectables.

Intravenous administration, preferably to a human subject, may be given either in the form of a bolus (injected all at once) (IV bolus) or intravenous infusion (IV infusion), for example, infused slowly through a vein of the subject into the plasma at a constant or zero-order rate. Preferably, intravenous administration is administered in the form of an intravenous infusion (IV infusion).

Such intravenous infusion, preferably to a human subject, may be provided as an isotonic solution. Such solutions generally have an osomolality of 250 to 375 mOsm/L. Preferred examples of isotonic solutions include normal saline (preferably ~ 0.9% sodium chloride), phosphate buffered saline, lactated Ringer's solution, ~ 5% dextrose in water (D5W), and Ringer's solution. For intravenous infusion, the isotonic solution preferably has a pH of from 5 to 8, such as from 6 to 8 or from 7.1 to 7.5.

For subcutaneous, intravenous, intramuscular, inhalation, or intraperitoneal administration, the compound (I), or a pharmaceutically acceptable salt, hydrate, or solvate thereof, may be provided as injectable doses in a pharmaceutically acceptable diluent together with a pharmaceutically acceptable excipient or carrier (which can be a sterile liquid or mixture of liquids).

For intramuscular, intraperitoneal, subcutaneous, inhalation, and intravenous use, compound (I), or a pharmaceutically acceptable salt, hydrate, or solvate thereof, will generally be provided in sterile aqueous solutions or suspensions, buffered to an appropriate pH and isotonicity.

Preferably, compound (I), or a pharmaceutically acceptable salt, hydrate, or solvate thereof, or a pharmaceutical composition formulated therefrom, is administered parenterally or by inhalation, more preferably, intravenously, and more preferably intravenously in the form of an intravenous infusion (IV infusion). Most preferably, compound (I), or a pharmaceutically acceptable salt, hydrate, or solvate thereof, its administered intravenously to human subjects, specifically in the form of an intravenous infusion (IV infusion).

Pharmaceutically acceptable excipients and carriers encompass all the foregoing and the like. The above considerations concerning effective formulations and administration procedures are well known in the art and are described in standard textbooks. See for example Remington: The Science and Practice of Pharmacy, 20th Edition (Lippincott, Williams and Wilkins), 2000; Lieberman et al., ed., Pharmaceutical Dosage Forms, Marcel Decker, New York, N. Y. (1980) and Kibbe et al., ed., Handbook of Pharmaceutical Excipients (3rd Edition), American Pharmaceutical Association, Washington (1999).

Suitable pharmaceutically acceptable carrier(s) or excipient(s) for use in pharmaceutical compositions of the present invention include isotonic solutions such as saline (preferably ~ 0.9% sodium chloride), phosphate buffered saline, lactated Ringer's solution, ~ 5% dextrose in water (D5W), and Ringer's solution, in addition to hydroxypropyl β cyclodextrin, and phosphate buffer. The isotonic solutions preferably have a pH of from 5 to 8, such as from 6 to 8 or from 7.1 to 7.5. The phosphate buffer may have a pH of 5 to 7, preferably 6.

For intravenous infusion to a human subject, the pharmaceutically acceptable carrier(s) or excipient(s) may be selected from saline (preferably ~ 0.9% sodium chloride), phosphate buffered saline, lactated Ringer's solution, ~ 5% dextrose in water (D5W), Ringer's solution, and phosphate buffer. The phosphate buffer may have a pH of 5 to 7, preferably 6. Preferably, for intravenous infusion to a human subject, the pharmaceutically acceptable carrier(s) or excipient(s) is phosphate buffer, preferably of pH 6.

For compound (I), or a pharmaceutically acceptable salt, hydrate, or solvate thereof, the dosage of the compound (I), or a pharmaceutically acceptable salt, hydrate, or solvate thereof administered will, of course, vary with the mode of administration, the treatment desired and the infection or disease indicated.

The size of the dose for therapeutic purposes of compound (I), or a pharmaceutically acceptable salt, hydrate, or solvate thereof, will naturally vary according to the nature and severity of the conditions, the age and sex of the subject and the mode of administration, according to well-known principles of medicine.

Dosage levels, dose frequency, and treatment durations of compound (I), or a pharmaceutically acceptable salt, hydrate, or solvate thereof, are expected to differ depending on the formulation, mode of administration, and clinical indication, age, and co-morbid medical conditions of the patient.

When the method of administration is intravenous infusion to a human subject, compound (I), or pharmaceutically acceptable salt, hydrate, or solvate thereof, may be administered, typically in a pharmaceutical composition according to the present invention, in a dose of from 50 to 6000 mg per day. Preferably, the compound (I), or pharmaceutically acceptable salt, hydrate, or solvate thereof, is administered at a dose of from 50 to 4000 mg per day, or 50 to 3000 mg per day, such as 50 to 2000 mg per day, or 100 to 2000 mg per day, such as 200 to 2000 mg per day, or 200 to 1750 mg per day, or 200 to 1500 mg per day, or 250 to 1000 mg per day. It will be appreciated that when the compound (I), or pharmaceutically acceptable salt, hydrate, or solvate thereof, is administered more than once per day, such as twice or three times per day as discussed below, the dose is divided according to frequency of administration per day.

When the method of administration is intravenous infusion to a human subject, at the doses stated above, administration of compound (I), or a pharmaceutically acceptable salt, hydrate, or solvate thereof may take place once (QD), twice (BID) or three (TID) times per day. Administration twice (BID) per day by intravenous infusion to a human subject includes two administrations per day separated by 1 to 12 hours, such as 1 to 8 hours, or 2 to 7 hours, or 2 to 6 hours, or 3 to 5 hours, such as 1 to 4 hours. Administration three (TID) times per day by intravenous infusion to a human subject includes three administrations per day separated by 2 to 7 hours, or 2 to 6 hours, or 3 to 5 hours, such as 1 to 4 hours. The number of hours is calculated from the time at which the first administration has begun. It is the period of time from which the first administration has begun, to the time at which the second administration is begun. It will further be appreciated that when administered twice or three times per day, the same dose or a different dose may be administered each time. Each administration of intravenous infusion may take place over a period of time of from 30 minutes to 6 hours, such as from 30 minutes to 4 hours, or from 30 minutes to 3 hours, or from 30 minutes to 2 hours, or from 30 minutes to 1 hour, or about 1 hour.

Preferably, the compound (I), or pharmaceutically acceptable salt, hydrate, or solvate thereof, is administered, by intravenous infusion to a human subject once per day (QD), twice per day (BID) 1 to 12 hours apart, such as 1 to 8 hours apart, or 2 to 6 hours apart, or 2 to 5 hours apart, or 3 to 5 hours apart, or 1 to 4 hours apart, or three times per day (TID) 2 to 7 hours apart, such as 2 to 6 hours apart, or 3 to 5 hours apart, such as 1 to 4 hours apart, at the doses stated above per day. More preferably, the compound (I), or pharmaceutically acceptable salt, hydrate, or solvate thereof, is administered by intravenous infusion to a human subject twice per day 1 to 12 hours apart, such as 1 to 8 hours apart, or 2 to 6 hours apart, or 2 to 5 hours apart, or 3 to 5 hours apart, or 1 to 4 hours apart, at the doses stated above per day.

When the method of administration is intravenous infusion to a human subject, at the doses and administration per day stated above, compound (I), or a pharmaceutically acceptable salt, hydrate, solvate, complex, or protected form thereof, is preferably used in the treatment of respiratory infections with Gram-negative bacteria of the order Enterobacterales and/or Gram-negative bacteria of the genus *Haemophilus,* and/or urinary tract infections (UTIs) with Gram-negative bacteria of the order Enterobacterales and/or Gram-negative bacteria of the genus *Haemophilus* (typically complicated UTIs).

When the method of administration is intravenous infusion to a human subject, at the doses and administration per day stated above, the course of treatment may last from 1 to 10 days, such as from 1 to 7 days, or from 1 to 5 days. By 'course of treatment' is meant the amount of time over which treatment is administrated on consecutive days.

Accordingly, a pharmaceutical composition according to the present invention comprising the compound (I), or a pharmaceutically acceptable salt, hydrate, or solvate thereof, may be administered by intravenous infusion to a human subject with the compound (I), or pharmaceutically acceptable salt, hydrate, or solvate thereof, at a dose of from 50 to 6000 mg per day, or 50 to 4000 mg per day, or 50 to 3000 mg per day, such as 50 to 2000 mg per day, or 100 to 2000 mg per day, such as 200 to 2000 mg per day, or 200 to 1750 mg per day, or 200 to 1500 mg per day, or 250 to 1000 mg per day, preferably once per day (QID), twice (BID) per day, or three times (TID) per day, more preferably once per day (QID), twice per day (BID) 1 to 12 hours apart, such as 1 to 8 hours apart, or 2 to 6 hours apart, or 2 to 5 hours apart, or 3 to 5 hours apart, or 1 to 4 hours apart, or three times (TID) per day 2 to 7 hours apart, such as 2 to 6 hours apart, or 3 to 5 hours apart, such as 1 to 4 hours apart, and more preferably twice per day 1 to 12 hours apart, such as 1 to 8 hours apart, or 2 to 6 hours apart, or 2 to 5 hours apart, or 3 to 5 hours apart, or 1 to 4 hours apart.

The compound (I), or a pharmaceutically acceptable salt, hydrate, or solvate thereof, may take any form. It may be synthetic, purified or isolated from natural sources using techniques described in the art.

Compound (I) may be obtained, stored and/or administered in the form of a pharmaceutically acceptable salt. Illustrative pharmaceutically acceptable salts are prepared from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, β-hydroxybutyric, galactaric and galacturonic acids.

Suitable pharmaceutically-acceptable base addition salts include metallic ion salts and organic ion salts. Metallic ion salts include, but are not limited to, appropriate alkali metal (group la) salts, alkaline earth metal (group Ila) salts and other physiologically acceptable metal ions. Such salts can be made from the ions of aluminium, calcium, lithium, magnesium, potassium, sodium and zinc. Organic salts can be made from tertiary amines and quaternary ammonium salts, including in part, trimethylamine, diethylamine, *N*, *N*'-dibenzylethylenediamine, chloroprocaine, choline, diethanolamine, ethylenediamine, meglumine (N-methylglucamine) and procaine. All of the above salts can be prepared by those skilled in the art by conventional means from the corresponding compound. Conventional procedures for the selection and preparation of suitable pharmaceutical formulations are described in, for example, "Pharmaceuticals - The Science of Dosage Form Designs", M. E. Aulton, Churchill Livingstone, 1988.

Compound (I), or a pharmaceutically acceptable salt, hydrate, or solvate thereof, may also be used in the *in vitro* inhibition of the growth of Gram-negative bacteria of the order Enterobacterales and/or Gram-negative bacteria of the genus *Haemophilus,* in particular carbapenem-resistant Enterobacterales (CRE) and extended spectrum β-lactamase (ESBL) Enterobacterales. Accordingly, the present invention further encompasses a method of *in vitro* inhibition of the growth of Gram-negative bacteria of the order Enterobacterales and/or Gram-negative bacteria of the genus *Haemophilus,* in particular carbapenem-resistant Enterobacterales (CRE) and extended spectrum β-lactamase (ESBL) Enterobacterales, using compound (I), or a pharmaceutically acceptable salt, hydrate, or solvate thereof.

Compound (I), or a pharmaceutically acceptable salt, hydrate, or solvate thereof, may be synthesised by any appropriate method.

Suitable methods for synthesising compound (I), or a pharmaceutically acceptable salt, hydrate, or solvate thereof include, but are not limited: a method corresponding to synthetic route 1 of WO 2019/086890.

Compound (I) may be synthesised according to the following method. This method represents a preferred method for the synthesis of compound (I), said method comprising the preferred connected steps detailed below. It will however be appreciated that, in the context of the present invention, these preferred steps are separable, and one or more of the steps may be substituted with any other suitable step identified by a skilled person. Where a Step X has an A and B option, for example Step 6A or Step 6B, either of Step XA or XB may be utilised as Step X, i.e. either of Step 6A or 6B may be utilised as Step 6.

### Step 1

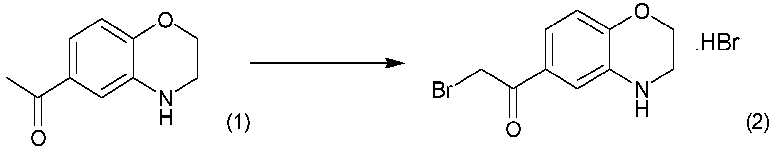

A 20 L four-necked round bottom flask equipped with mechanical stirrer was charged with 1-(3,4-dihydro-2H-1,4-benzoxazin-6-yl)ethan-1-one (1) (300 g, 1.69 mol) at room temperature. A solution of HBr in glacial acetic acid (33% w/w, 2.10 L, 7 V) was added dropwise at 10°C ideally maintaining the internal temperature below ~12°C. To the resulting reaction mixture was added a solution of bromine (86.7 mL, 1.69 mol) in glacial acetic acid (450 mL, 1.5 V with respect to starting material) at 10°C (internal temperature) slowly (~ over 90 min) such that internal temperature was maintained below 10°C. The reaction was monitored by LCMS. Following complete addition of a solution of bromine in glacial acetic, the crude LCMS confirmed ~10% unconsumed starting material (1-(3,4-dihydro-2H-1,4-benzoxazin-6-yl)ethan-1-one (1)) and ~30% conversion to desired mono-brominated product (2) along with ~24% di-brominated by-product. The cooling bath was removed and the reaction mixture was gradually warmed to 25°C (internal temperature) over approximately 1h. The resulting reaction mixture was further stirred at 40°C (internal temperature) for 1.5 h [visible precipitation was observed, the crude LCMS confirmed unconsumed starting material (1) (~4%) along with desired mono-brominated product (2) (55%) and di-brominated by-product (∼18%)]. The reaction mixture was allowed to cool to 10 °C, diluted with MTBE (4.9 L, 16 V) and stirred for 1h. The resulting precipitate was filtered, washed with MTBE (600 mL) and dried under reduced pressure to afford 340 g (mixture of mono and dibrominated product) as an off white solid. The LCMS of obtained solid confirmed a mixture of 2-bromo-1-(3,4-dihydro-2H-1,4-benzoxazin-6-yl)ethan-1-one.HBr (2) (~87%, mono brominated), 2,2-dibromo-1-(3,4-dihydro-2H-benzo[b][1,4]oxazin-6-yl)ethan-1-one (~5%, dibrominated by-product) and unconsumed 1-(3,4-dihydro-2H-1,4-benzoxazin-6-yl)ethan-1-one (1) (~1%). This mixture was used in the next step without further purification. The isolated yield calculated on the basis of mono brominated product (2) observed by LCMS was found to be 52%. MS (ESI+) for CHNOS m/z 255.96 [M+H]⁺, ¹H NMR (400 MHz, DMSO-*d6*): *δ* 7.19-7.28 (m, 2H), 6.77 (d, *J =* 8.4 Hz, 1H), 4.74 (s, 2H), 4.31 (t, *J =* 4.4 Hz, 2H) and 3.32 (t, *J* = 4.4 Hz, 2H).

### Step 2

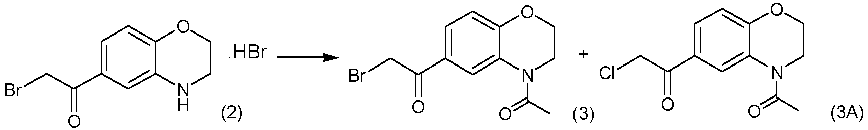

A 10 L four-necked round bottom flask equipped with mechanical stirrer was charged with THF (3.50 L, 7V) and 2-bromo-1-(3,4-dihydro-2H-1,4-benzoxazin-6-yl)ethan-1-one.HBr (2) (500 g, 1.48 mol) at 20°C. To the resulting reaction mixture was added acetyl chloride (476 mL, 6.68 mol) dropwise maintaining the temperature at 20°C. A 2°C increase in the reaction temperature was observed, during the addition of acetyl chloride to the reaction mixture. The reaction mixture was further stirred at 30°C for 16h. The reaction was monitored by LCMS. The crude LCMS confirmed formation of mixture of 1-(4-acetyl-3,4-dihydro-2H-benzo[b][1,4]oxazin-6-yl)-2-bromoethan-1-one & 1-(4-acetyl-3,4-dihydro-2H-benzo[b][1,4]oxazin-6-yl)-2-chloroethan-1-one in 1:1 ratio . The reaction mixture was cooled to ~10° C and basified to pH ~8.0 with 10% aqueous solution of K₂CO₃ (5.0 L) and extracted with ethyl acetate (3 X 5L). The combined organic layer was washed with brine (5.0 L), and concentrated under reduce pressure to afford crude product as a brown liquid. The brown liquid was cooled to 10°C, diluted with hexane (4.0 L) and stirred at 10°C for 1h. The resulting precipitate was filtered, washed with hexane (1.0 L) and dried under vacuum to afford a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (4) as a light brown solid (387 g, 92% pure by LCMS), which was used in next step without further purification. MS (ESI+) for CHNOS *m*/*z* 297.99 [M+H]⁺ & 254.05 [M+H]⁺.

### Step 3

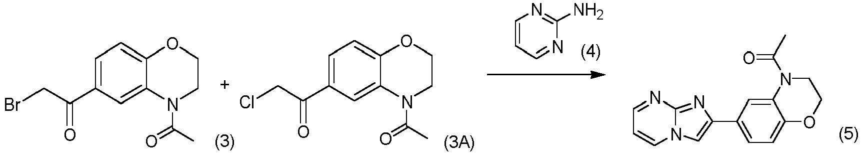

A 5.0 L four-necked round bottom flask equipped with mechanical stirrer was charged with EtOH (2.0 L, 3 V), a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A) (685 g, 2.30 mol, 92% pure by LCMS) followed by addition of pyrimidin-2-amine (4) (546 g, 5.74 mol) at 20°C. The reaction mixture was further stirred and refluxed for 3h. The reaction was monitored with LCMS. The reaction mixture was allowed to cool to room temperature (~ 20°C) and the resulting precipitated solid was filtered, washed with EtOH (2 X 250 mL) and dried under vacuum to afford 1 -(6-(imidazo[1,2-a]pyrimidin-2-yl)-2,3-dihydro-4H-benzo[b][1,4]oxazin-4-yl)ethan-1-one (5) as an off white solid (418 g). MS (ESI+) for CHNOS *m*/*z* 295.06 [M+H]⁺. 1H NMR (400 MHz, DMSO-d*6* + *d*-TFA): *δ* 9.27 (d, *J* = 6.40 Hz, 1H), 8.97 -9.02 (m, 1H), 8.65 (s, 1H), 8.44 (bs, 1H), 7.63-7.69 (m, 2H), 7.10 (d, *J* = 8.40 Hz, 1H), 4.33-4.36 (m, 2H), 3.89-3.93 (m, 2H), 2.32 (s, 3H). The regioisomeric structure was confirmed by nOe.

### Step 4

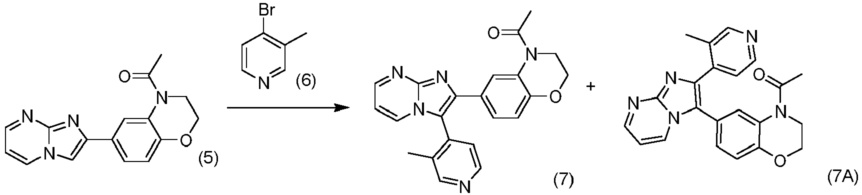

A 10 L four-necked round bottom flask equipped with mechanical stirrer was charged with N,N-dimethylacetamide (2.0 L), 1-(6-(imidazo[1,2-a]pyrimidin-2-yl)-2,3-dihydro-4H-benzo[b][1,4]oxazin-4-yl)ethan-1-one (5) (250 g, 849 mmol), 2,2-dimethylpropanoic acid (34.7 g, 340 mmol), potassium carbonate (587 g, 4.25 mol) and 4-bromo-3-methylpyridine (6) (264 g, 1.44 mol) at 20° C under N₂ atmosphere. The resulting reaction mixture was purged with N₂ (g) for 30 min followed by addition of Pd(OAc)₂ (19.1 g, 84.9 mmol) and PCy₃·HBF₄ (31.2 g, 84.9 mmol) under N₂ atmosphere. The reaction mixture was again purged with N₂ (g) for additional 15 min. The resulting reaction mixture was further stirred at 125°C for 7h. The reaction was monitored with LCMS. The crude LCMS confirmed the formation of regioisomeric mixture of desired product in 98:2 regioisomeric ratio. The reaction mixture was filtered through celite bed (1.3 cm height & 25 cm diameter). The celite bed was washed with 10% MeOH in DCM (15 L). The filtrate was evaporated under reduced pressure to afford the crude residue as a brown liquid. The brown liquid was stirred in heptane (3 x 6.0 L) for 10 h to remove excess of DMA. The heptane/DMA mixture was decanted (traces of desired product was observed in the decanted fraction) to afford waxy solid. The waxy solid was further stirred in MTBE (3.0 L) for 15 min. The resulting precipitate was filtered and washed with MTBE (2.0 L) to afford the desired product as a brown solid. The brown solid was passed through a small silica plug eluting with 10% MeOH in DCM (~35 L). The obtained solvent fraction was concentrated to 1/10 of its original volume. The concentrated fraction was diluted with MTBE (200 mL) and stirred for 30 min. The resulting precipitate was filtered, washed with MTBE (1.0 L) and dried under vacuum to afford regioisomeric mixture of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (220 g) as a light brown solid. Yield: 66% (97% by LCMS, 98:2 regioisomeric ratio). MS (ESI+) for CHNOS m/z 386.18 [M+1]⁺. 1H NMR (400 MHz, DMSO-d*6* + *d*-TFA): *δ* 9.14 (s, 1H), 9.01-9.04 (m, 2H), 8.86 (d, *J* = 6.7 Hz, 1H), 8.23 (d, *J* = 5.8 Hz, 1H), 8.01 (bs, 1H), 7.55-7.58 (m, 1H), 7.41(d, *J* = 8.3 Hz, 1H), 7.05 (d, *J* = 8.6 Hz, 1H), 4.09-4.33 (m, 2H), 3.71-3.92 (m, 2H), 2.17 (s, 3H), 2.11 (bs, 3H).

### Step 5A

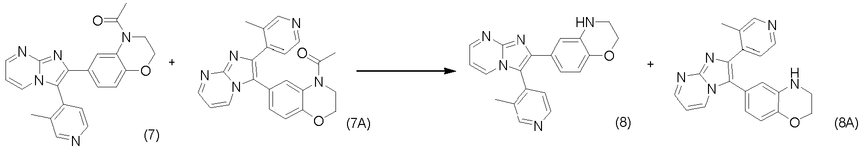

To a solution of a regioisomeric mixture (97:3) of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (24.0 g, 62.3 mmol) in methanol (150 mL) was added 6.0 N aqueous HCl (62 mL) at rt. The reaction mixture was stirred at 90°C for 16 h. The reaction mixture was allowed to cool to room temperature and concentrated to 1/4th of its original volume. The concentrated reaction mixture was basified to pH 8-9 with saturated aq. NaHCO₃ solution and extracted with 10% MeOH in DCM (3 x 250 mL). The organic layer was washed with brine (300 mL), dried over (Na₂SO₄), filtered and concentrated to 1/10th of its original volume under reduced pressure. The concentrated mixture was diluted with heptane (100 mL) and stirred for 30 min. The resulting precipitate was filtered, washed with heptane (100 mL) and dried under reduced pressure to obtain mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) **(**20.0 g) as a yellow solid. Yield: 71% (mixture of two regioisomers in 2:1 regioisomeric ratio, 89% by LCMS). The LCMS showed two peaks with desired mass 56% and 33% respectively. (ESI+) for CHNOS *m*/*z* 344.20 [M+H]⁺.

### Or Step 5B

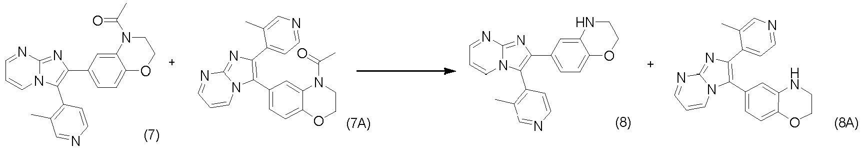

A suspension of a regioisomeric mixture (98:2) of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (10.0 g, 25.9 mmol) in 2.0 M aq. solution of NaOH (52 mL, 104 mmol) was heated at 100°C for 24 h. The reaction was monitored with the LCMS. After completion of the reaction, the reaction mixture was allowed to cool to room temperature and filtered. The filtered cake was washed with the water (~500 mL), dried to obtain mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) as a light brown solid. Yield: 8.1 g, 90% (mixture of two regioisomers in 98:2 regioisomeric ratio, 98.7% by LCMS). MS (ESI+) for CHNOS *m*/*z* 344.20 [M+H]⁺, ¹H NMR (400 MHz, DMSO-d₆): *δ* 8.69 (s, 1H), 8.54-8.62 (m, 2H), 8.25 (d, *J* = 6.8 Hz, 1H), 7.46 (d, *J =* 4.8 Hz, 1H), 6.96-7.03 (m, 2H), 6.46-6.55 (m, 2H), 5.87 (s, 1H), 4.11 (t, *J* = 4.0 Hz, 2H), 3.25 (bs, 2H), 1.94 (s, 3H).

### Step 6A (HMPA mediated amino acid coupling)

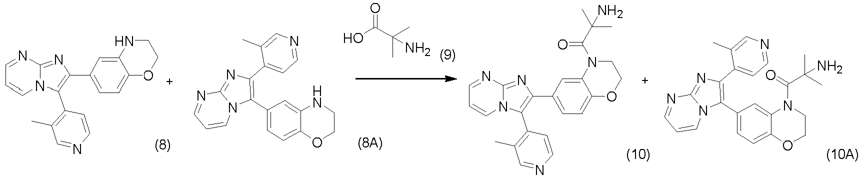

To a suspension of 2-amino-2-methylpropanoic acid (9) (12 g, 116 mmol) in HMPA (84 mL, 7 V) was added a solution of thionyl chloride (9.29 g, 128 mmol) in ACN (8.4 mL) slowly at 3°C (outer temperature was 0°C). The suspension becomes clear after 10 min of the complete addition of solution of thionyl chloride in ACN. The resulting reaction mixture was stirred at 0°C for 20 min followed by portion wise addition of a regioisomeric mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (8 g, 23.3 mmol) at 0°C. The reaction mixture was allowed to warm to room temperature and stirred for 16 h. The reaction was monitored by LCMS. After the completion of the reaction, the reaction mixture was diluted with EtOAc (300 mL). The resulting precipitate was filtered and washed with EtOAc (250 mL). The solid was dissolved in water (50 mL) and neutralized with saturated sodium bicarbonate solution up to the pH of 8 and extracted with 10% MeOH in DCM (4 X 100 mL), concentrated under reduced pressure to 1/10th of original reaction mixture volume. The crude mixture was diluted with MTBE (50 mL) and stirred for 15 min. The resulting precipitate was filtered, washed with MTBE (25 mL) and dried under vacuum to afford regioisomeric mixture of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (9.1 g) as a brown solid. Yield: 66% (97% by LCMS, 98:2 regioisomeric ratio). MS (ESI+) for CHNOS m/z 429.16 [M+1]⁺ , ¹H NMR (400 MHz, DMSO-ds): *δ* 8.69 (s, 1H), 8.54-8.62 (m, 2H), 8.29 (d, *J* = 5.2 Hz, 1H), 8.12 (d, *J* = 1.6 Hz, 1H), 7.46 (d, *J* = 4.8 Hz, 1H), 6.98-7.05 (m, 2H), 6.77 (d, *J* = 8.4 Hz, 1H), 4.45- 4.67 (m, 2H), 4.30 (bs, 2H), 2.12 (bs, 2H), 1.97 (s, 3H), 1.34 (s, 6H).

### Or Step 6B (DMPU mediated amino acid coupling)

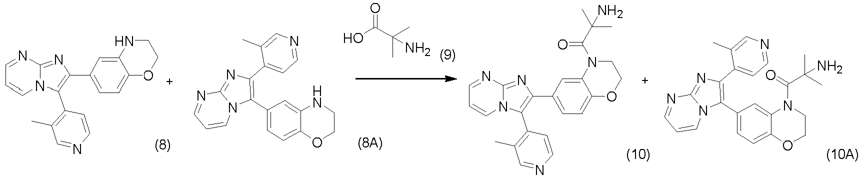

To a solution of 2-amino-2-methylpropanoic acid (9) (2.0 g, 19.4 mmol) in DMPU (14 mL) was added a solution of thionyl chloride (1.55 mL, 21.3 mmol) in ACN (1.4 mL) slowly at 4°C (outer temperature was 0°C). The resulting reaction mixture was stirred at 0°C for 20 min followed by portion wise addition of a regioisomeric mixture (98:1) of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (1.33 g, 3.88 mmol) at 0°C. The reaction mixture was allowed to warm to room temperature and stirred for 16 h. After the completion of the reaction (LCMS monitoring), the reaction mixture was diluted with EtOAc (50 mL). The resulting precipitate was filtered and washed with EtOAc (50 mL). The solid was dissolved in water (100 mL), basified to pH ~8.0 with saturated sodium bicarbonate solution and extracted with 10% MeOH in DCM (3 X 100 mL), dried (Na₂SO₄) and concentrated under reduced pressure to obtain the crude residue. The crude residue was further stirred in the MTBE (20 mL), filtered, washed with MTBE (20 mL) and dried under vacuum to afford regioisomeric mixture of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (1.3 g, 98.7% by LCMS, 98:2 regioisomeric ratio) as a brown solid, which was further used in next step without further purification. MS (ESI+) for CHNOS m/z 429.16 [M+1]⁺; ¹H NMR (400 MHz, DMSO-d₆): *δ* 8.69 (s, 1H), 8.54-8.62 (m, 2H), 8.29 (d, *J* = 5.2 Hz, 1H), 8.12 (d, *J* = 1.6 Hz, 1H), 7.46 (d, *J* = 4.8 Hz, 1H), 6.98-7.05 (m, 2H), 6.77 (d, *J* = 8.4 Hz, 1H), 4.45- 4.67 (m, 2H), 4.30 (bs, 2H), 2.12 (bs, 2H), 1.97 (s, 3H), 1.34 (s, 6H).

### Step 7

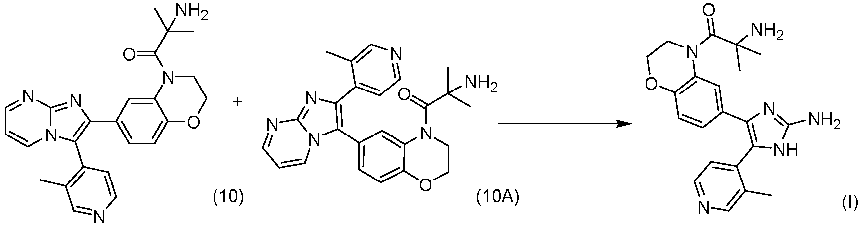

To a suspension of a regioisomeric mixture (98:2) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (8.6 g, 93% by LCMS, 20.1 mmol) in isopropyl alcohol (60 mL) was added hydroxylamine hydrochloride (3.49 g, 50.2 mmol) at room temperature. The resulting suspension was stirred at 70°C for 22h. The reaction was monitored by LCMS. The crude LCMS showed formation of desired product along with the traces of de-amidated by-product (1-2% by crude LCMS) and unreacted starting material (2-3% by LCMS). The resulting suspension was allowed to cool to room temperature, diluted with acetone (100 mL) and stirred for 1h. The reaction mixture was concentrated to 20% of original volume and precipitation was filtered, washed with acetone (200 mL) and dried under vacuum to afford crude compound (I) as an HCl salt. The obtained solid was dissolved in the water (10 mL), basified up to pH-8 by saturated solution of NaHCO₃ and extracted with 10% MeOH in DCM (5 x 100 mL). The combined organic layer was dried over Na₂SO₄, filtered and concentrated under vacuum to obtain the crude residue. The crude residue was stirred in the EtOH (10 mL). The crude product solubilizes in EtOH and after stirring for 1-2 h, again precipitation was observed. The resulting precipitate was filtered and washed with EtOH (10 mL). The precipitate thus obtained was again stirred in EtOH (10 mL) , filtered, followed by the washing with 2% MeOH in DCM (15 mL) and dried under vacuum to afford 2-amino-1-(6-(2-amino-5-(3-methylpyridin-4-yl)-1H-imidazol-4-yl)-2,3-dihydro-4H-benzo[b][1,4]oxazin-4-yl)-2-methylpropan-1-one (compound (I)) as a light yellow fluffy solid. Yield: 3.5 g, 44% (98.3% by LCMS). MS (ESI+) for CHNOS m/z 392.8 [M+H]⁺. ¹H NMR (400 MHz, DMSO-*d₆* + d-TFA): *δ* 8.78-8.84 (m, 2H), 7.93 (d, *J* = 6.1 Hz, 1 H), 7.44 (d, *J* = 2.1 Hz, 1 H), 7.09 (dd, *J* = 2.1, 8.6 Hz, 1 H), 6.97 (d, *J* = 8.6 Hz, 1 H), 4.34 (bs, 2H), 3.97 (bs, 2H), 2.16 (s, 3H), 1.60 (s, 6H).

Preferably Step 5B is selected.

It will be appreciated that a person skilled in the art will be readily able to synthesise pharmaceutically acceptable salts, hydrates, or solvates of compound (I), typically using any of the syntheses disclosed herein to form compound (I), followed by known additional step(s) required to form a pharmaceutically acceptable salt, hydrate, or solvate thereof.

The invention will now be described with reference to specific examples.

### Examples

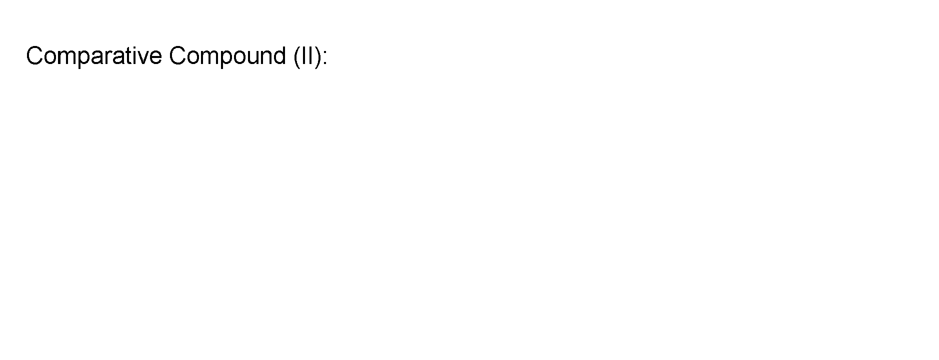

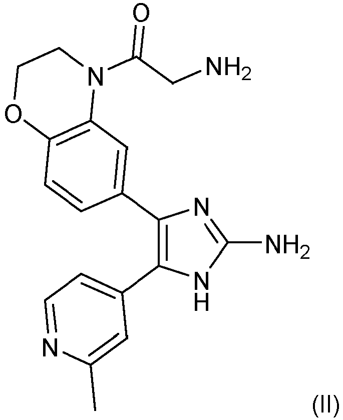

was synthesised by a method corresponding to Synthetic Route 1 described in WO 2019/086890. Comparative Compound (II) may also be synthesised by modification of the method outlined for compound (I) above. Such modification includes selection of the appropriate pyridyl starting material and the use glycine as the amino acid. 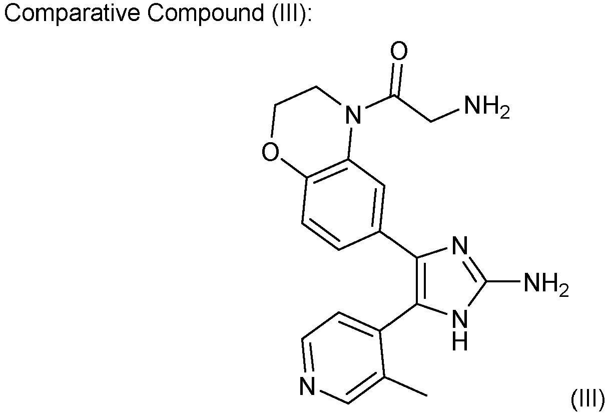 was synthesised by a method corresponding to Synthetic Route 1 described in WO 2019/086890. Comparative Compound (III) may also be synthesised by modification of the method outlined for compound (I) above. Such modification includes selection of the appropriate pyridyl starting material and the use glycine as the amino acid.

Compound (I) and Comparative Compounds (II) and (III) were subject to the following analysis relating to minimum inhibitory concentration (MIC) and minimum inhibitory concentration 90 values (MIC₉₀).

### Antibacterial Susceptibility

Minimum Inhibitory Concentrations (MICs) versus *Escherichia coli* (NCTC 13441) and *Klebsiella pneumoniae* (NCTC 13438) (planktonic bacteria) were determined by the broth microdilution procedure in line with the guidelines of the Clinical and Laboratory Standards Institute (Clinical and Laboratory Standards Institute. Methods for Dilution Antimicrobial Susceptibility Tests for Bacteria That Grow Aerobically; Approved Standard-Eleventh Edition, CLSI document M07, 11 January 2018). The broth dilution method involved a two-fold serial dilution of compounds in 96-well microtitre plates, giving a final concentration range of 0.39-200 µM and a maximum final concentration of 2% DMSO. The bacterial strains tested were *Escherichia coli* (NCTC 13441) and *Klebsiella pneumoniae* (NCTC 13438). Strains were grown in cation-adjusted Müller-Hinton broth or on Luria Bertani agar at 37°C in an ambient atmosphere. The MIC (µM) was determined as the lowest concentration of compound that inhibits growth following a 20-24 hour incubation period. The results are set out in Table 1.

**Table 1**

| | *Escherichia coli* (NCTC 13441) (µM) | *Klebsiella pneumoniae* (NCTC 13438) (µM) |
|---|---|---|
| Compound (I) | ≤0.39 | 1.56-3.13 |

Minimum Inhibitory Concentrations (MICs) versus *Proteus mirabilis (DSM 4479)* were determined for compound (I) and comparative compounds (II) and (III) using the methodology outlined above. The results are set out in Table 2. Compound (I) shows a superior potency versus comparative compounds (II) and (III).

**Table 2**

| | *Proteus mirabilis* (µM) |
|---|---|
| Compound (I) | 25 |
| Comparative Compound (II) | >200 |
| Comparative Compound (III) | >200 |

Minimum Inhibitory Concentration (MIC₉₀) versus 100 isolates of *Escherichia coli* and *Klebsiella pneumonia* were determined. The Minimum Inhibitory Concentration at which 90% of the isolates (strains) are inhibited (MIC₉₀) were measured by broth microdilution in line with EUCAST susceptibility testing standards (www.eucast.org). Bacterial inocula were prepared at ca 1 x 10^6 CFU/mL by diluting 100-fold a 0.5 McFarland suspension. Antibacterial panels containing 50 µl of antibacterial solutions at 2 x the final concentrations were diluted 2-fold with 50 µl of inoculum to give a final inoculum of ca 5 x 10^5 CFU/ml and desired test concentrations of antibacterial agents (0.03 - 64 µg/mL). The plates were incubated according to the guidelines of the Clinical and Laboratory Standards Institute (Clinical and Laboratory Standards Institute. Methods for Dilution Antimicrobial Susceptibility Tests for Bacteria That Grow Aerobically; Approved Standard-Eleventh Edition, CLSI document M07, 11 January 2018). The MIC₉₀ (µg/ml) is the MIC value at which greater than or equal to 90% of the isolates (strains) within a test population are inhibited. The results are set out in Table 3.

**Table 3**

| | *Escherichia coli* (100 isolates) | | *Klebsiella pneumonia* (100 isolates) | |
|---|---|---|---|---|
| | MIC₉₀ (µg/mL) | Range (µg/mL) | MIC₉₀ (µg/mL) | Range (µg/mL) |
| Compound (I) | 1.00 | 0.12 - 4.00 | 2.00 | 0.25-8.00 |

Minimum Inhibitory Concentrations (MICs) versus *Proteus spp.* and *Providencia spp*. were determined for compound (I) and comparative compound (III) using the methodology outlined above for the 100 isolates of *Escherichia coli* and *Klebsiella pneumonia.* The results are set out in Table 4. Compound (I) shows a superior potency versus comparative compound (III).

**Table 4**

| | *Proteus spp.* (10 isolates) MIC Range (µg/mL) | *Providencia spp.* (10 isolates) MIC Range (µg/mL) |
|---|---|---|
| Compound (I) | 8-32 | 0.5-16 |
| Comparative Compound (III) | 64->64 | 1->64 |

Compound (I) and Comparative Compounds (II) and (III) were subject to the following analysis relating to lipophilicity and volume of distribution (Vss).

### Lipophilicity

Log P values of compound (I) and comparative compound (III) were measured using the SiriusT3 instrument from Sirius Analytical by performing an acid-based titration and measuring the shift in pKa when the sample solution is in contact with an immiscible solvent (octanol). Log P for the comparative compound (II) was calculated using Marvin (physico-chemical calculation software). Results are shown in Table 5. The lipophilicity data for compound (I) and comparative compounds (II) and (III) was collected in separate experiments carried out on different days, and compared in Table 5.

**Table 5**

| | Compound (I) | Comparative Compound (II) | Comparative Compound (III) |
|---|---|---|---|
| Log P | 1.6 | 0.07 | 1.03 |

### Volume of Distribution

The volume of distribution (Vss) of compound (I) and comparative compounds (II) and (III) was determined from analysis of plasma concentration time profiles obtained from rodent species (mice), following 5 mg/kg IV bolus administration respectively. The volume of distribution was obtained by analysing the rodent plasma profiles for compound (I) and comparative compounds (II) and (III) using a 2 compartment IV bolus model in PK Solver (Excel). The outputted Vss values are shown in Table 6. The volume of distribution data for compound (I) and comparative compound (II) was collected in separate experiments carried out on different days, and compared in Table 6.

**Table 6**

| | Compound (I) | Comparative Compound (II) | Comparative Compound (III) |
|---|---|---|---|
| Vss (L/kg) | 19.7 | 7.7 | 7.9 |

### Discussion of Results

Without being bound by theory, the present inventors consider that above results relating to lipophilicity and volume of distribution demonstrate that compound (I), or a pharmaceutically acceptable salt, hydrate, or solvate thereof, may be advantageously used in the treatment of infection with, or disease caused or exacerbated by, Gram-negative bacteria of the order Enterobacterales and/or Gram-negative bacteria of the genus *Haemophilus,* in particular in the treatment of multi-site infections with Gram-negative bacteria of the order Enterobacterales and/or Gram-negative bacteria of the genus *Haemophilus.* The present inventors consider that the above results demonstrate that compound (I), or a pharmaceutically acceptable salt, hydrate, or solvate thereof, has the ability to be distributed effectively around a subject's body to all infection sites at sufficient concentration so as to enable effective treatment at every site. As well as sufficient amounts of the compound being available to exert a pharmacological effect, there is a balance to be maintained between the distribution of the compound from the blood plasma being sufficiently rapid such that the drug can act quickly against the infection, and avoiding overly rapid release which would prevent the drug reaching all possible infection sites in sufficient concentration.

Such a balance is considered to be demonstrated by the above results, which are discussed below in more detail.

A Log P value of a compound provides an indication of polarity and thus, the ability of the compound to reach the target tissue in a subject, i.e. the ease at which the drug can cross membrane barriers and be distributed around a subject's body to possible intracellular infection sites.

From Table 5, it can be seen that compound (I) of the present invention has a higher Log P value and thus lower polarity relative to both comparative compounds (II) and (III), indicating improved tissue distribution of compound (I) compared to the more polar comparative compounds (II) and (III). The present inventors consider that this improved tissue distribution of compound (I) enables the compound to be distributed around a subject's body to all possible infection sites, with enough drug reaching each of the infection sites to enable treatment. If the drug is distributed less effectively, such as will be seen for comparative compound (II) and (III) based on the Log P values of Table 5, there will be insufficient drug concentration across the infection sites and treatment of the infection hindered. As discussed above, there is a balance to be achieved regarding the efficiency at which the drug is distributed around the subject's body. The Log P value of compound (I) indicates that it is distributed to the multi-sites of infection and provides appropriate drug concentration at all of these infection sites versus comparative compounds (II) and (III).

Volume distribution (Vss) provides an indication of the degree to which the compound is distributed in a subject's body tissue rather than the blood plasma, i.e. the propensity of the compound to leave the blood plasma and be distributed in the subject's body tissue.

From Table 6, it can be seen that compound (I) has surprising and unexpectedly better tissue penetration characteristics compared to comparative compound (II) and comparative compound (III), which are both structurally very similar to compound (I).

The present inventors have determined that compound (I) demonstrates good permeability. The lipophilicity and permeability of compound (I) allows the compound to reach and penetrate the target tissue in a subject. The surprising and unexpectedly better tissue distribution and penetration characteristics of compound (I) versus comparative compounds (II) and (III) result from the advantageous lipophilicity and permeability of compound (I).

### In Vitro Safety Pharmacology Study

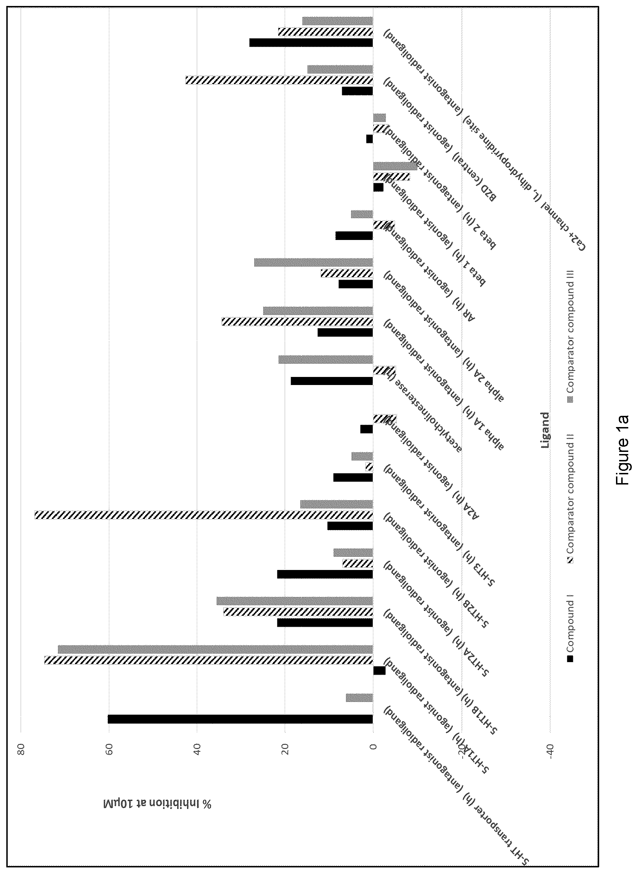

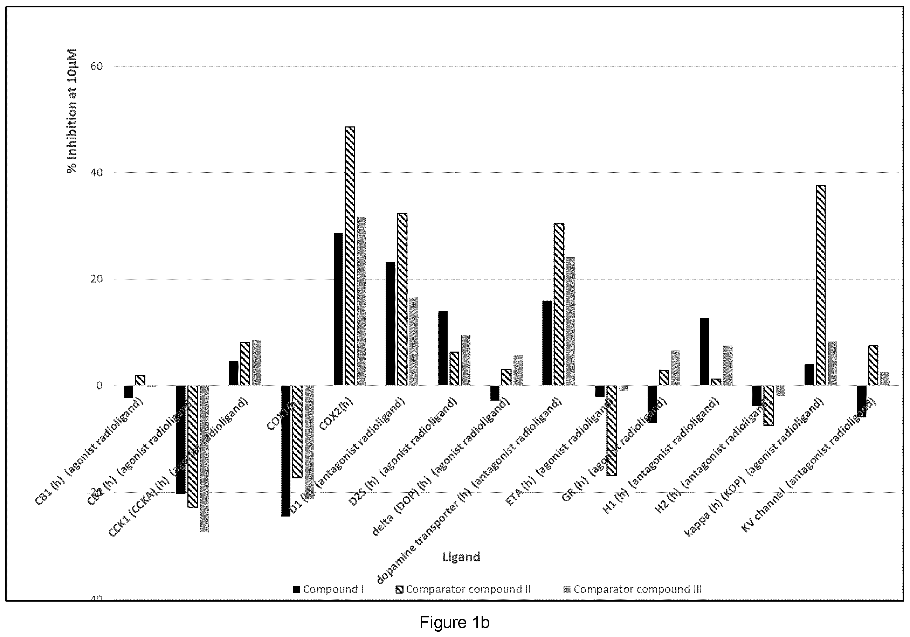

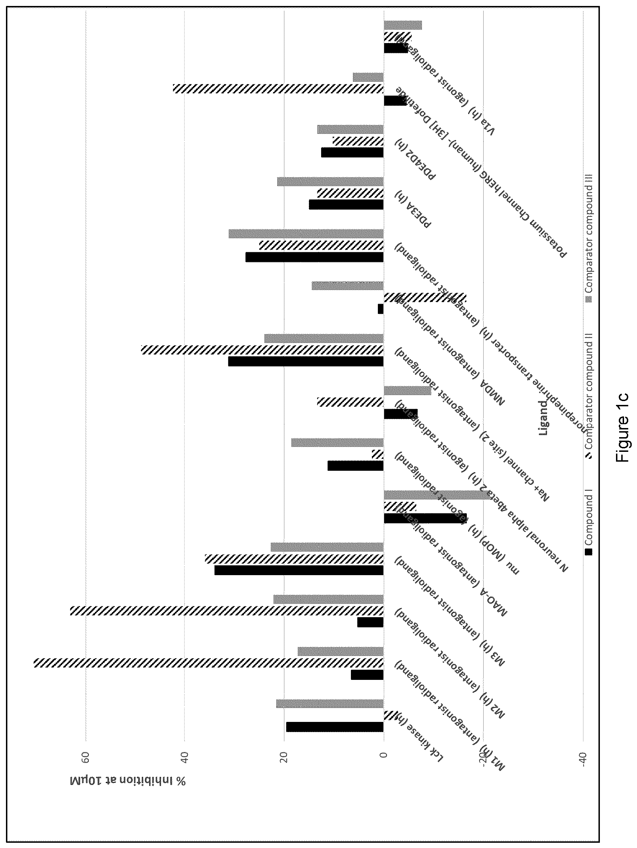

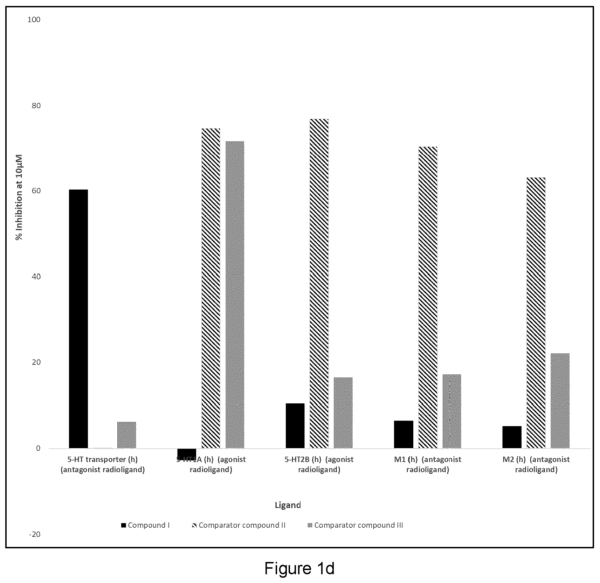

*In vitro* pharmacology binding assays were carried out for compound (I), comparative compound (II) and comparative compound (III) to evaluate the percentage of inhibition against 44 ligands (including receptors, transporters, ion channels, enzymes and kinases) at a concentration of 10µM of compound (I), comparative compound (II) or comparative compound (III). Compound binding was calculated as a percentage of inhibition of the binding of a radioactively labelled ligand specific for each target. Results showing an inhibition higher than 50% are considered to represent discernible effects of compound (I), comparative compound (II) or comparative compound (III). Results for all 44 ligands tested are shown in Figures 1a, 1b, and 1c, whilst Figure 1d shows only the results showing an inhibition higher than 50%. The *in vitro* pharmacology binding assays for compound (I) and comparative compounds (II) and (III) was carried out separately over different days, and compared in Figures 1a, 1b, 1c, and 1d.

From Figures 1a, 1b, 1c, and 1d, it can be seen that compound (I) has a different CEREP profile than both of comparative compounds (II) and (III). Compound (I) demonstrated inhibition higher than 50% for only a single ligand, i.e. fewer (and different) ligands than comparative compounds (II) and/or (III).

### Proof-of-concept Studies

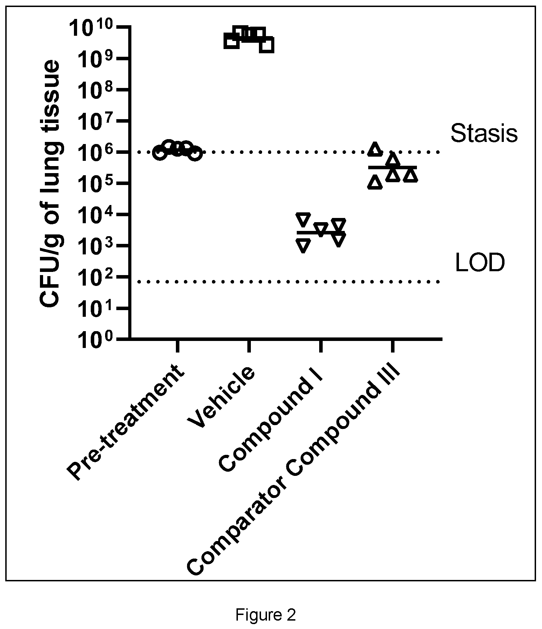

Compound (I) and comparative compound (III) were evaluated in proof-of-concept studies in murine models for the treatment of a respiratory infection with Gram-negative bacteria of the order Enterobacterales, a bacteraemia or bloodstream infection with Gram-negative bacteria of the order Enterobacterales, and a urinary tract infection with Gram-negative bacteria of the order Enterobacterales. Results are shown in Figures 2, 3, and 4a-c. Each dot, triangle or square indicator represents a single mouse. LOD refers to the limit of detection of the bacteria.

Stasis shows the level of bacteria (colony-forming unit (CFU)) in the subject pre-treatment. Each of the proof-of-concept studies was carried out separately. By vehicle is meant pharmaceutically acceptable carrier or excipient.

For the proof-of-concept study for the treatment of a respiratory infection caused or exacerbated by Gram-negative bacteria of the order Enterobacterales, CD-1 mice were infected with *Klebsiella pneumoniae* ATCC 43816. Two hours after infection ('pre-treatment' in Figure 2), a vehicle (20% hydroxypropyl β cyclodextrin) containing no compound (I) or comparative compound (III), or a vehicle (20% hydroxypropyl β cyclodextrin) containing compound (I) or comparative compound (III), was administered by IV bolus at 20mg/kg, TID (8 hrs apart), over one day.

From Figure 2, it can be seen that, at the end of the study (26 h from initial infection: 'compound (I)' and 'comparative compound (III)' in Figure 2) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. In fact, the CFU value for comparative compound (III) in Figure 2 remained relatively close to the stasis level, i.e. the instance in which no killing of the bacteria has occurred. From Figure 2, it can be seen that at the end of the study (26h from initial infection), compound (I) reduced bacterial burden in the lung by 6.24 log compared to vehicle, whereas comparative compound (III) only demonstrated a 4.16 log reduction compared to vehicle. Compound (I) is thus 2 log more efficacious than comparative compound (III) in the treatment of a respiratory infection with *Klebsiella pneumoniae*, in particular multidrug-resistant *Klebsiella pneumonia.*

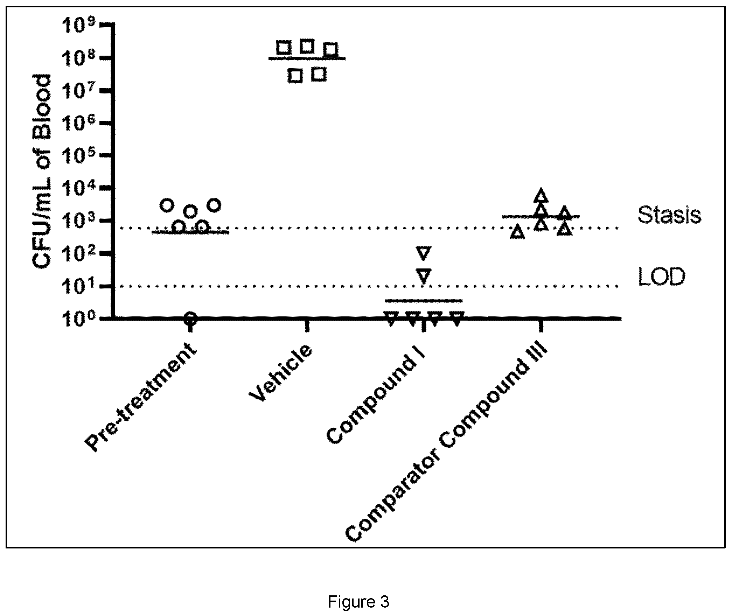

For the proof-of-concept study for the treatment of a bacteraemia or bloodstream infection with Gram-negative bacteria of the order Enterobacterales, CD-1 mice were infected with human urine isolate *E*. *coli* BAA-2469 (NDM-1 positive). One hour after infection ('pre-treatment' in Figure 3), a vehicle (20% hydroxypropyl β cyclodextrin) containing no compound (I) or comparative compound (III), or a vehicle (20% hydroxypropyl β cyclodextrin) containing compound (I) or comparative compound (III), was administered by as a single dose IV bolus at 20 mg/kg.

From Figure 3, it can be seen that, at the end of the study (9h from initial infection: 'compound (I)' and 'comparative compound (III)' in Figure 3) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. In fact, the CFU value for comparative compound (III) in Figure 3 remained on or above the stasis level, i.e. the instance in which no killing of the bacteria has occurred. From Figure 3, it can be seen that at the end of the study (9h after initial infection), compound (I) reduced the bacterial burden in the blood below the limit of detection (LOD), with a 7.43 log reduction compared to vehicle. In contrast, comparative compound (III) only demonstrated a 4.86 log reduction compared to vehicle. Compound (I) is thus 2.5 log more efficacious than comparative compound (III) in the treatment of a bacteraemia or bloodstream infection with *Escherichia Coli,* in particular multidrug-resistant *Escherichia Coli.*

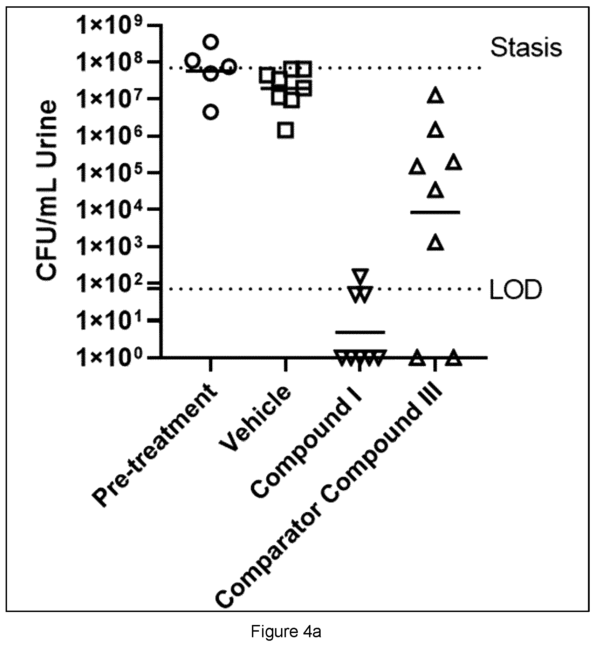

For the proof-of-concept study for the treatment of a urinary tract infection with Enterobacterales, female C3H/HeN mice were infected with *E*. *coli* UTI89. Twenty-four hours after infection ('pre-treatment' in Figures 4a-c), a vehicle (20% hydroxypropyl β cyclodextrin) containing no compound (I) or comparative compound (III), or a vehicle (20% hydroxypropyl β cyclodextrin) containing compound (I) or comparative compound (III), was administered by IV bolus of 20 mg/kg, TID (8 hrs apart), over 3 days.

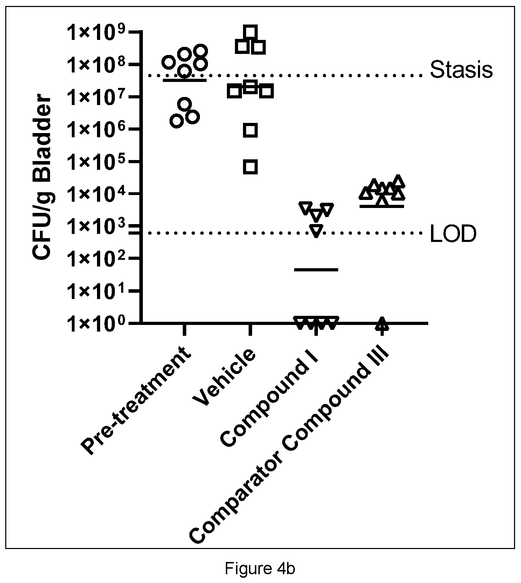

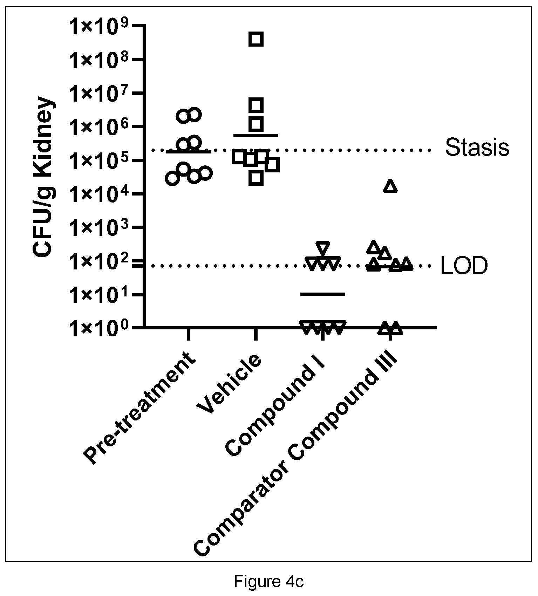

As urinary tract infections may affect the bladder and kidneys, bacterial burden in each of the urine, bladder and kidney was assessed (Figures 4a-c respectively). From Figures 4a and 4b, it can be seen that, at the end of the study (96h after initial infection: 'compound (I)' and 'comparative compound (III)' in Figures 4a and 4b) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. From Figure 4c, it can be seen that, at the end of the study (96h after initial infection: 'compound (I)' and 'comparative compound (III)' in Figures 4c) the colony-forming unit (CFU) was also reduced for compound (I) with respect to comparative compound (III), i.e. the bacterial burden has been reduced through treatment with compound (I) versus comparative compound (III), compared to pre-treatment and vehicle. From Figure 4a, it can be seen that at the end of the study (96h after initial infection), compound (I) reduced the bacterial burden in the urine to below the limit of detection (LOD), with a 6.59 log reduction compared to vehicle. In contrast, comparative compound (III) only demonstrated a reduction of 3.36 log compared to vehicle. Compound (I) is thus 3 log more efficacious than comparative compound (III) in reducing the CFU in the urine, and thus facilitates the treatment of urinary tract infections (UTIs) with *Escherichia coli.* From Figure 4b, it can be seen that at the end of the study (96h after initial infection) compound (I) reduced the bacterial burden in the bladder by 5.67 log compared to vehicle. In comparison, comparative compound (III) only demonstrated a reduction of 3.71 log compared to vehicle. Compound (I) is thus 3 log more efficacious than comparative compound (III) in reducing the CFU in the bladder, and thus facilitates the treatment of urinary tract infections (UTIs) with *Escherichia coli.* From Figure 4c, it can be see that at the end of the study (96h after initial infection) compound (I) reduced the bacterial burden in the kidney by 4.73 log compared to vehicle.

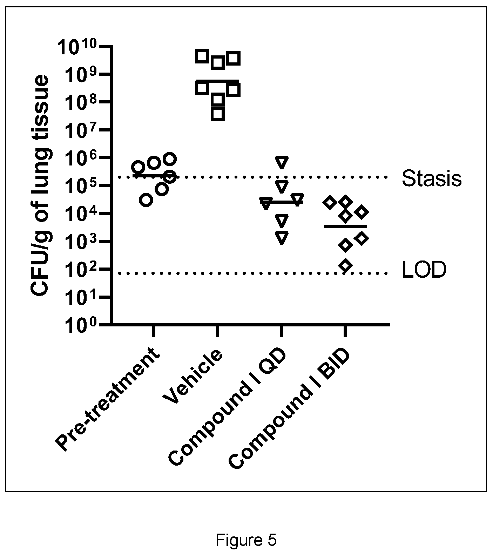

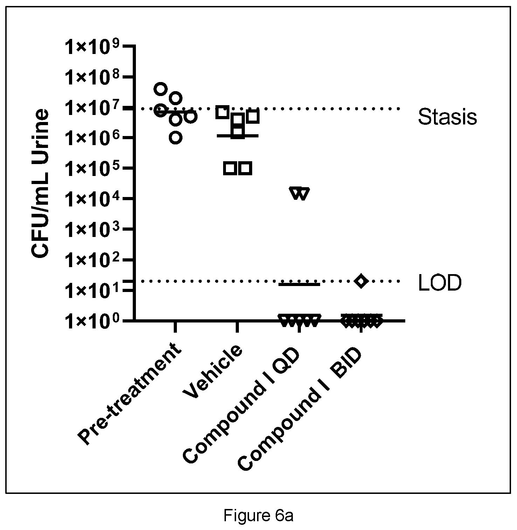

Compound (I) was evaluated in further proof-of-concept studies in murine models for the treatment of a respiratory infection with Gram-negative bacteria of the order Enterobacterales, and a urinary tract infection with Gram-negative bacteria of the order Enterobacterales. Results are shown in Figures 5 and 6a-c. Each dot, triangle or square indicator represents a single mouse. LOD refers to the limit of detection of the bacteria. Stasis shows the level of bacteria (colony-forming unit (CFU)) in the subject pre-treatment. Each of the proof-of-concept studies was carried out separately. By vehicle is meant pharmaceutically acceptable excipient or carrier.

For the further proof-of-concept study for the treatment of a respiratory infection caused or exacerbated by Gram-negative bacteria of the order Enterobacterales, male CD-1 mice were infected with *Klebsiella pneumoniae* ATCC 43816.

Two hours after infection ('pre-treatment' in Figure 5), mice were either treated with a vehicle control (phosphate buffer) containing no compound (I), or a vehicle (phosphate buffer) containing compound (I), administered by intravenous infusion for 1 hour (QD) with a dose of 20 mg/kg of compound (I). For the mice that received the vehicle control containing no compound (I), and some mice who received the vehicle containing compound (I), an additional 1-hour continuous intravenous infusion was administered 3 hours after the time at which the first administration begun (BID, 3 hrs apart).

From Figure 5, it can be seen that, at the end of the study (26 h from initial infection: 'compound (I) QD' and 'compound (I) BID' in Figure 5) the colony-forming unit (CFU) was reduced for compound (I), i.e. the bacterial burden has been reduced through treatment with compound (I), compared to pre-treatment and vehicle ('vehicle BID' in Figure 5). This was especially seen for compound (I) when administered twice (BID, 3 hrs apart) (P value of <0.0001 compared to both pre-treatment and vehicle). The P value for compound (I) when administered once (QD) was 0.0042 compared to pre-treatment and <0.0001 compared to vehicle). From Figure 5, it can be seen that at the end of the study (26h from initial infection), compound (I) reduced bacterial burden in the lung by 4.35 and 5.20 log (for once (QD) and twice (BID, 3 hrs apart) administration respectively) compared to vehicle, and 0.95 and 1.80 log (for once (QD) and twice (BID, 3 hrs apart) administration respectively) compared to pre-treatment (stasis levels). Compound (I) is thus effective in the treatment of a respiratory infection with *Klebsiella pneumoniae*, in particular multidrug-resistant *Klebsiella pneumonia.*

For the further proof-of-concept study for the treatment of a urinary tract infection with Enterobacterales, female C3H/HeN mice were infected with *E*. *coli* UTI89.

Twenty-four hours after infection ('pre-treatment' in Figures 6a-c), mice were either treated with a vehicle control (20% hydroxypropyl β cyclodextrin) containing no compound (I), or a vehicle (20% hydroxypropyl β cyclodextrin) containing compound (I), administered by intravenous infusion for 1 hour (QD) with a dose of 20 mg/kg of compound (I). For some mice who received vehicle containing compound (I), an additional 1-hour continuous intravenous infusion was administered 5 hours after the time at which the first administration begun (BID, 5 hrs apart). The once or twice daily administration was continued for three days. For QD, administration took place between 24 to 25 hours, 48 to 49 hours, and 72 to 73 hours after infection, and for BID, administration took place between 24 to 25 hours, 29 to 30 hours, 48 to 49 hours, 53 to 54 hours, 72 to 73 hours and 77 to 78 hours after infection.

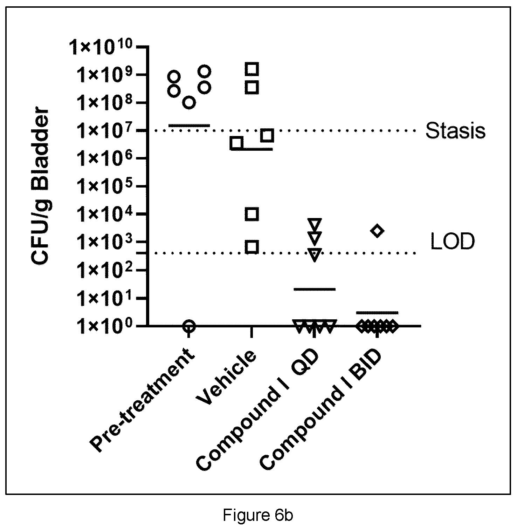

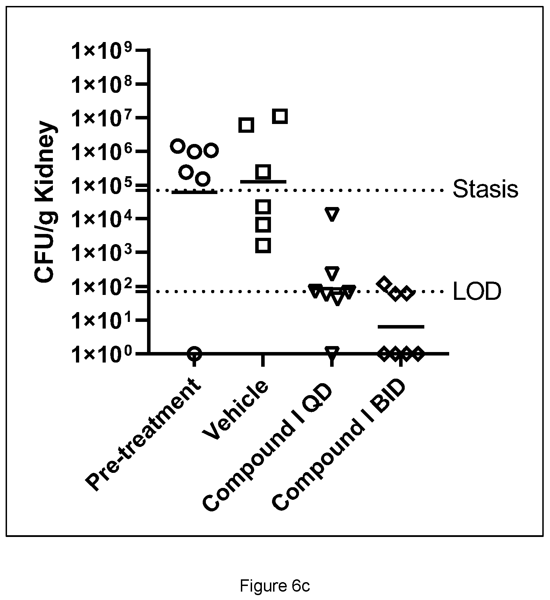

As urinary tract infections may affect the bladder and kidneys, bacterial burden in each of the urine, bladder and kidney was assessed (Figures 6a-c respectively). From Figures 6a, 6b and 6c, it can be seen that, at the end of the study (96h after initial infection: 'compound (I) QD' and 'compound (I) BID' in Figures 6a, 6b and 6c) the colony-forming unit (CFU) was significantly reduced for compound (I), i.e. the bacterial burden has been significantly reduced through treatment with compound (I), compared to pre-treatment and vehicle. This was especially seen for compound (I) when administered twice daily (BID, 5 hrs apart). From Figure 6a, it can be seen that at the end of the study (96h after initial infection), compound (I) reduced the bacterial burden in the urine to below the limit of detection (LOD) for both QD and BID daily administration, with a 4.86 and 5.87 log reduction (for once (QD) and twice (BID, 5 hrs apart) daily administration respectively) compared to vehicle. The P value for compound (I), whether administered once (QD) or twice (BID, 5 hrs apart) was <0.0001 compared to vehicle. Compound (I) is thus effective in reducing CFU in the urine, and thus facilitates the treatment of urinary tract infections (UTls) with *Escherichia coli.* From Figure 6b, it can be seen that at the end of the study (96h after initial infection) compound (I) reduced the bacterial burden in the bladder to below the limit of detection (LOD) for both QD and BID daily administration, with a 5.01 and 5.84 log reduction (for once (QD) and twice (BID, 5 hrs apart) daily administration respectively) compared to vehicle.

The P value for administration once per day (QD) was 0.001 compared to vehicle, and for administration twice per day (BID, 5 hrs apart) was 0.0002. Compound (I) is thus effective in reducing the CFU in the bladder, and thus facilitates the treatment of urinary tract infections (UTIs) with *Escherichia coli.* From Figure 6c, it can be see that at the end of the study (96h after initial infection) compound (I) reduced the bacterial burden in the kidney to below the limit of detection (LOD) for BID administration, with 3.17 and 4.3 log reduction (for once (QD) and twice (BID, 5 hrs apart) daily administration respectively) compared to vehicle. The P value for administration once per day (QD) was 0.0007 compared to vehicle, and for administration twice per day (BID, 5 hrs apart) was <0.0001. Compound (I) is thus effective in reducing the CFU in the kidney, and thus facilitates the treatment of urinary tract infections (UTIs) with *Escherichia coli.*

## Claims

1. A compound (I): 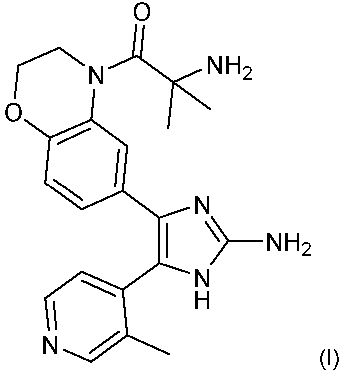 or a pharmaceutically acceptable salt, hydrate, or solvate thereof.

2. The compound (I) according to claim 1, or pharmaceutically acceptable salt, hydrate, or solvate thereof, for use in therapy or prophylaxis of an infection with, or disease caused or exacerbated by, Gram-negative bacteria of the order Enterobacterales and/or Gram-negative bacteria of the genus *Haemophilus.*

3. The compound (I) according to claim 1, or a pharmaceutically acceptable salt, hydrate, or solvate thereof, for use in the treatment of infection with, or disease caused or exacerbated by, Gram-negative bacteria of the order Enterobacterales and/or Gram-negative bacteria of the genus *Haemophilus.*

4. The compound (I) for use according to claim 2 or 3, or a pharmaceutically acceptable salt, hydrate, or solvate thereof, wherein the infection with, or disease caused or exacerbated by, Gram-negative bacteria of the order Enterobacterales and/or Gram-negative bacteria of the genus *Haemophilus,* is one or more of: a bacteraemia or bloodstream infection with Gram-negative bacteria of the order Enterobacterales and/or Gram-negative bacteria of the genus *Haemophilus,* a respiratory infection with Gram-negative bacteria of the order Enterobacterales and/or Gram-negative bacteria of the genus *Haemophilus,* a urinary tract infection (UTI) with Gram-negative bacteria of the order Enterobacterales and/or Gram-negative bacteria of the genus *Haemophilus,* pyelonephritis with Gram-negative bacteria of the order Enterobacterales and/or Gram-negative bacteria of the genus *Haemophilus,* and an intra-abdominal infection with Gram-negative bacteria of the order Enterobacterales and/or Gram-negative bacteria of the genus *Haemophilus,* preferably wherein the infection with, or disease caused or exacerbated by, Gram-negative bacteria of the order Enterobacterales and/or Gram-negative bacteria of the genus *Haemophilus,* is one or more of: a bacteraemia or bloodstream infection with Gram-negative bacteria of the order Enterobacterales and/or Gram-negative bacteria of the genus *Haemophilus,* a respiratory infection with Gram-negative bacteria of the order Enterobacterales and/or Gram-negative bacteria of the genus *Haemophilus,* and a urinary tract infection (UTI) with Gram-negative bacteria of the order Enterobacterales and/or Gram-negative bacteria of the genus *Haemophilus,* and more preferably wherein the infection with, or disease caused or exacerbated by, Gram-negative bacteria of the order Enterobacterales and/or Gram-negative bacteria of the genus *Haemophilus,* is one or more of: a respiratory infection with Gram-negative bacteria of the order Enterobacterales and/or Gram-negative bacteria of the genus *Haemophilus,* and a urinary tract infection (UTI) with Gram-negative bacteria of the order Enterobacterales and/or Gram-negative bacteria of the genus *Haemophilus*; and/or wherein the infection with, or disease caused or exacerbated by, Gram-negative bacteria of the order Enterobacterales and/or Gram-negative bacteria of the genus *Haemophilus,* is a multi-site infection.

5. The compound (I) for use according to claim 2, 3 or 4, or a pharmaceutically acceptable salt, hydrate, or solvate thereof, wherein the compound (I) or a pharmaceutically acceptable salt, hydrate, or solvate thereof is administered parenterally, preferably intravenously, and more preferably, by an intravenous infusion (IV infusion).

6. The compound (I) for use according to any of claims 2 to 5, or a pharmaceutically acceptable salt, hydrate, or solvate thereof, wherein the Gram-negative bacteria of the order Enterobacterales and/or Gram-negative bacteria of the genus *Haemophilus* is a carbapenem-resistant Enterobacterales or an extended spectrum β-lactamase Enterobacterales; and/or wherein the Gram-negative bacteria is of the order Enterobacterales; and/or wherein the Gram-negative bacteria of the order Enterobacterales is selected from the Enterobacterales genera of *Arsenophonus, Atlantibacter, Biostraticola, Brenneria, Buchnera, Budvicia, Buttiauxella, Cedecea, Chania, Citrobacter, Cosenzaea, Cronobacter, Dickeya, Edwardsiella, Enterobacillus, Enterobacter, Erwinia, Escherichia, Ewingella, Franconibacter, Gibbsiella, Hafnia, Izhakiella, Kosakonia, Klebsiella, Kluyvera, Leclercia, Lelliottia, Leminorella, Levinea, Lonsdalea, Mangrovibacter, Moellerella, Morganella, Obesumbacterium, Pantoea, Pectobacterium, Phaseolibacter, Photorhabdus, Plesiomonas, Pluralibacter, Pragia, Proteus, Providencia, Pseudocitrobacter, Rahnella, Raoultella, Rosenbergiella, Rouxiella, Saccharobacter, Salmonella, Samsonia, Serratia, Shigella, Shimwellia, Siccibacter, Sodalis, Tatumella, Thorsellia, Trabulsiella, Wigglesworthia, Xenorhabdus, Yersinia* and *Yokenella*, preferably wherein the Gram-negatvie bacteria of the order Enterobacterales is selected from *Cedecea, Citrobacter, Erwinia, Escherichia, Enterobacter, Klebsiella, Kluyvera, Plesiomonas*, *Proteus*, *Providencia*, *Raoultella*, *Salmonella*, *Serratia*, *Shigella*, and *Yersinia*, preferably wherein the Gram-negatvie bacteria of the order Enterobacterales is selected from *Erwinia*, *Escherichia*, *Enterobacter*, *Klebsiella*, *Proteus*, *Salmonella*, *Serratia*, *Shigella*, and *Yersinia*, preferably wherein the Gram-negative bacteria of the order Enterobacterales is selected from the Enterobacterales genera of *Enterobacter, Escherichia* and *Klebsiella*, and more preferably wherein the Gram-negative bacteria of the order Enterobacterales is selected from the Enterobacterales genera of *Escherichia* and *Klebsiella*; and/or wherein the Gram-negative bacteria of the order Enterobacterales is selected from *Enterobacter spp.*, *Escherichia coli* and *Klebsiella pneumonia*, preferably *Escherichia coli* and *Klebsiella pneumonia.*

7. A method of *in vitro* inhibition of the growth of Gram-negative bacteria of the order Enterobacterales and/or Gram-negative bacteria of the genus *Haemophilus* using the compound (I) according to claim 1, or a pharmaceutically acceptable salt, hydrate, or solvate thereof.

8. A compound (I): 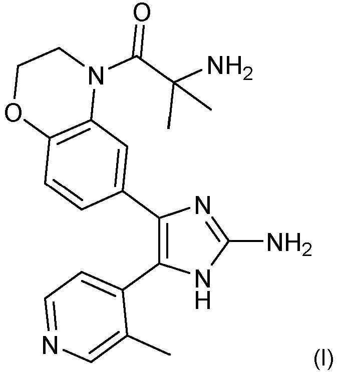 or a pharmaceutically acceptable salt, hydrate, or solvate thereof, formulated together with a pharmaceutically acceptable excipient(s) or carrier(s); preferably wherein the pharmaceutically acceptable excipient(s) or carrier(s) is a pharmaceutically acceptable excipient(s) or carrier(s) suitable for an intravenous infusion to a human subject.

9. A pharmaceutical composition comprising the compound (I) according to claim 1, or a pharmaceutically acceptable salt, hydrate, or solvate thereof, and a pharmaceutically acceptable excipient(s) or carrier(s); preferably wherein the pharmaceutically acceptable excipient(s) or carrier(s) is a pharmaceutically acceptable excipient(s) or carrier(s) suitable for an intravenous infusion to a human subject; and/or wherein the compound (I), or pharmaceutically acceptable salt, hydrate, or solvate thereof, is present in an amount of from 50 to 6000 mg, preferably 50 to 4000 mg, such as 50 to 3000 mg, such as 50 to 2000 mg, such as 100 to 2000 mg, such as 200 to 2000 mg, such as 200 to 1750 mg, such as 200 to 1500 mg, such as 250 to 1000 mg.

10. A pharmaceutical composition for use in the treatment of infection with, or disease caused or exacerbated by, Gram-negative bacteria of the order Enterobacterales and/or Gram-negative bacteria of the genus *Haemophilus,* the pharmaceutical composition comprising the compound (I) according to claim 1, or a pharmaceutically acceptable salt, hydrate, or solvate thereof, and a pharmaceutically acceptable excipient(s) or carrier(s), preferably wherein the pharmaceutical composition is administered by intravenous infusion to a human subject.

11. A pharmaceutical composition for use in the treatment of infection with, or disease caused or exacerbated by, Gram-negative bacteria of the order Enterobacterales and/or Gram-negative bacteria of the genus *Haemophilus,* the pharmaceutical composition comprising the compound (I) according to claim 1, or a pharmaceutically acceptable salt, hydrate, or solvate thereof, and a pharmaceutically acceptable excipient(s) or carrier(s), wherein the pharmaceutical composition is administered by intravenous infusion to a human subject, with the compound (I), or pharmaceutically acceptable salt, hydrate, or solvate thereof, at a dose of 50 to 6000 mg per day.

12. The pharmaceutical composition for use according to claim 10, wherein the compound (I), or pharmaceutically acceptable salt, hydrate, or solvate thereof, is administered to a human subject at a dose of from 50 to 6000 mg per day.

13. The pharmaceutical composition for use according to claim 10, 11 or 12, wherein the compound (I), or pharmaceutically acceptable salt, hydrate, or solvate thereof, is administered to a human subject at a dose of from 50 to 4000 mg per day, such as 50 to 3000 mg per day, such as 50 to 2000 mg per day, such as 100 to 2000 mg per day, such as 200 to 2000 mg per day, such as 200 to 1750 mg per day, such as 200 to 1500 mg per day, such as 250 to 1000 mg per day.

14. The pharmaceutical composition for use according to any of claims 11 to 13, wherein the pharmaceutical composition is administered by intravenous infusion to a human subject once per day, twice per day, or three times per day, preferably twice per day; and/or wherein when the pharmaceutical composition is administered twice per day, this is 1 to 12 hours apart, such as 1 to 8 hours apart, or 2 to 6 hours apart, or 2 to 5 hours apart, or 3 to 5 hours apart, or 1 to 4 hours apart.

15. The pharmaceutical composition for use according to any of claims 11 to 14, wherein the pharmaceutical composition is administered by intravenous infusion to a human subject, and is for use in the treatment of respiratory infections with Gram-negative bacteria of the order Enterobacterales and/or Gram-negative bacteria of the genus *Haemophilus,* and urinary tract infections (UTIs) with Gram-negative bacteria of the order Enterobacterales and/or Gram-negative bacteria of the genus *Haemophilus* (typically complicated UTIs); and/or wherein the pharmaceutical composition is administered by intravenous infusion to a human subject and the treatment of infection with, or disease caused or exacerbated by, Gram-negative bacteria of the order Enterobacterales and/or Gram-negative bacteria of the genus *Haemophilus* involves a course of treatment of 1 to 10 days, such as from 1 to 7 days, or 1 to 5 days.

## Patentansprüche

1. Verbindung (I): 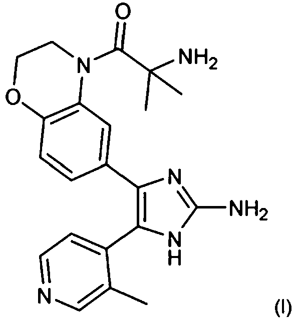 oder ein pharmazeutisch annehmbares Salz, Hydrat oder Solvat davon.

2. Verbindung (I) nach Anspruch 1, oder pharmazeutisch annehmbares Salz, Hydrat oder Solvat davon zur Therapie oder Prophylaxe einer Infektion oder Krankheit, die durch gramnegative Bakterien der Ordnung Enterobacterales und/oder gramnegative Bakterien der Gattung Haemophilus verursacht oder verschlimmert werden.

3. Verbindung (I), nach Anspruch 1, oder ein pharmazeutisch annehmbares Salz, Hydrat oder Solvat davon zur Behandlung von Infektionen oder Krankheiten, die durch gramnegative Bakterien der Ordnung Enterobacterales und/oder gramnegative Bakterien der Gattung Haemophilus verursacht oder verschlimmert werden.

4. Verbindung (I), zur Verwendung nach Anspruch 2 oder 3, oder ein pharmazeutisch annehmbares Salz, Hydrat oder Solvat davon, wobei die Infektion mit gramnegativen Bakterien der Ordnung Enterobacterales und/oder gramnegativen Bakterien der Gattung Haemophilus oder die Krankheit, die dadurch verursacht oder verschlimmert wird, eine oder mehrere der folgenden sind: eine Bakteriämie oder Blutstrominfektion mit gramnegativen Bakterien der Ordnung Enterobacterales und/oder gramnegativen Bakterien der Gattung Haemophilus, eine Atemwegsinfektion mit gramnegativen Bakterien der Ordnung Enterobacterales und/oder gramnegativen Bakterien der Gattung Haemophilus, eine Harnwegsinfektion (HWI) mit gramnegativen Bakterien der Ordnung Enterobacterales und/oder gramnegativen Bakterien der Gattung Haemophilus, Pyelonephritis mit gramnegativen Bakterien der Ordnung Enterobacterales und/oder gramnegativen Bakterien der Gattung Haemophilus und eine intraabdominale Infektion mit gramnegativen Bakterien der Ordnung Enterobacterales und/oder gramnegativen Bakterien der Gattung Haemophilus, wobei die Infektion mit gramnegativen Bakterien der Ordnung Enterobacterales und/oder gramnegativen Bakterien der Gattung Haemophilus oder die Krankheit, die dadurch verursacht oder verschlimmert wird, bevorzugt eine oder mehrere der folgenden sind: eine Bakteriämie oder Blutstrominfektion mit gramnegativen Bakterien der Ordnung Enterobacterales und/oder gramnegativen Bakterien der Gattung Haemophilus, eine Atemwegsinfektion mit gramnegativen Bakterien der Ordnung Enterobacterales und/oder gramnegativen Bakterien der Gattung Haemophilus und eine Harnwegsinfektion (HWI) mit gramnegativen Bakterien der Ordnung Enterobacterales und/oder gramnegativen Bakterien der Gattung Haemophilus, und wobei die Infektion mit gramnegativen Bakterien der Ordnung Enterobacterales und/oder gramnegativen Bakterien der Gattung Haemophilus oder die Krankheit, die dadurch verursacht oder verschlimmert wird, noch bevorzugter eine oder mehrere der folgenden sind: eine Atemwegsinfektion mit gramnegativen Bakterien der Ordnung Enterobacterales und/oder gramnegativen Bakterien der Gattung Haemophilus und eine Harnwegsinfektion (HWI) mit gramnegativen Bakterien der Ordnung Enterobacterales und/oder gramnegativen Bakterien der Gattung Haemophilus; und/oder wobei die Infektion mit gramnegativen Bakterien der Ordnung Enterobacterales und/oder gramnegativen Bakterien der Gattung Haemophilus oder die Krankheit, die dadurch verursacht oder verschlimmert wird, eine Multi-Site-Infektion ist.

5. Verbindung (I), zur Verwendung nach Anspruch 2, 3 oder 4, oder ein pharmazeutisch annehmbares Salz, Hydrat oder Solvat davon, wobei die Verbindung (I) oder ein pharmazeutisch annehmbares Salz, Hydrat oder Solvat davon parenteral, bevorzugt intravenös, und noch bevorzugter durch eine intravenöse Infusion (Infusion IV), verabreicht wird.

6. Verbindung (I), zur Verwendung nach einem der Ansprüche 2 bis 5, oder ein pharmazeutisch annehmbares Salz, Hydrat oder Solvat davon, wobei die gramnegativen Bakterien der Ordnung Enterobacterales und/oder die gramnegativen Bakterien der Gattung Haemophilus ein carbapenemresistentes Enterobacterales oder ein Enterobacterales mit Beta-Laktamasen mit erweitertem Spektrum sind; und/oder wobei die gramnegativen Bakterien der Ordnung Enterobacterales sind; und/oder wobei die gramnegativen Bakterien der Gattung Enterobacterales ausgewählt sind aus den Genera von Arsenophonus, Atlantibacter, Biostraticola, Brenneria, Buchnera, Budvicia, Buttiauxella, Cedecea, Chania, Citrobacter, Cosenzaea, Cronobacter, Dickeya, Edwardsiella, Enterobacillus, Enterobacter, Erwinia, Escherichia, Ewingella, Franconibacter, Gibbsiella, Hafnia, Izhakiella, Kosakonia, Klebsiella, Kluyvera, Leclercia, Lelliottia, Leminorella, Levinea, Lonsdalea, Mangrovibacter, Moellerella, Morganella, Obesumbacterium, Pantoea, Pectobacterium, Phaseolibacter, Photorhabdus, Plesiomonas, Pluralibacter, Pragia, Proteus, Providencia, Pseudocitrobacter, Rahnella, Raoultella, Rosenbergiella, Rouxiella, Saccharobacter, Salmonella, Samsonia, Serratia, Shigella, Shimwellia, Siccibacter, Sodalis, Tatumella, Thorsellia, Trabulsiella, Wigglesworthia, Xenorhabdus, Yersinia und Yokenella, wobei bevorzugt die gramnegativen Bakterien der Ordnung Enterobacterales ausgewählt sind aus Cedecea, Citrobacter, Erwinia, Escherichia, Enterobacter, Klebsiella, Kluyvera, Plesiomonas, Proteus, Providencia, Raoultella, Salmonella, Serratia, Shigella und Yersinia, wobei bevorzugt die gramnegativen Bakterien der Ordnung Enterobacterales ausgewählt sind aus Erwinia, Escherichia, Enterobacter, Klebsiella, Proteus, Salmonella, Serratia, Shigella und Yersinia, wobei bevorzugt die gramnegativen Bakterien der Ordnung Enterobacterales ausgewählt sind aus den Genera der Enterobacterales von Enterobacter, Escherichia und Klebsiella, und wobei die gramnegativen Bakterien der Ordnung Enterobacterales noch bevorzugter ausgewählt sind aus den Genera der Enterobacterales von Escherichia und Klebsiella; und/oder wobei die gramnegativen Bakterien der Ordnung Enterobacterales ausgewählt sind aus Enterobacter spp., Escherichia coli und Klebsiella pneumoniae, bevorzugt Escherichia coli und Klebsiella pneumoniae.

7. Verfahren zur In-vitro-Inhibierung des Wachstums von gramnegativen Bakterien der Ordnung Enterobacterales und/oder gramnegativen Bakterien der Gattung Haemophilus unter Verwendung der Verbindung (I) nach Anspruch 1, oder ein pharmazeutisch annehmbares Salz, Hydrat oder Solvat davon.

8. Verbindung (I): 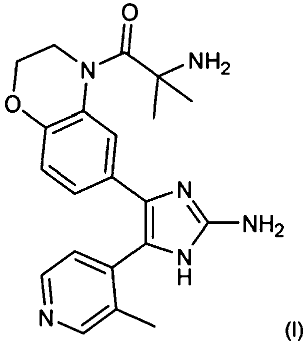 oder ein pharmazeutisch annehmbares Salz, Hydrat oder Solvat davon, zusammen mit einem oder mehreren pharmazeutisch annehmbaren Hilfsstoffen oder Träger(n) formuliert; wobei der eine oder die mehreren pharmazeutisch annehmbaren Hilfsstoffe oder Träger bevorzugt ein oder mehrere pharmazeutisch annehmbare Hilfsstoffe oder Träger sind, der/die für eine intravenöse Infusion an einem Menschen geeignet ist/sind.

9. Pharmazeutische Zusammensetzung, die Verbindung (I) nach Anspruch 1 umfassend, oder ein pharmazeutisch annehmbares Salz, Hydrat oder Solvat davon und einen oder mehrere pharmazeutisch annehmbare Hilfsstoffe oder Träger; wobei bevorzugt der eine oder die mehreren pharmazeutisch annehmbaren Hilfsstoffe oder Träger ein oder mehrere pharmazeutisch annehmbare Hilfsstoffe oder Träger ist/sind, der/die für eine intravenöse Infusion an einem Menschen geeignet ist/sind; und/oder wobei die Verbindung (I) oder das pharmazeutisch annehmbare Salz, Hydrat oder Solvat davon in einer Menge von 50 bis 6000 mg vorhanden ist, bevorzugt 50 bis 4000 mg, z. B. 50 bis 3000 mg, z. B. 50 bis 2000 mg, z. B. 100 bis 2000 mg, z. B. 200 bis 2000 mg, z. B. 200 bis 1750 mg, z. B. 200 bis 1500 mg, z. B. 250 bis 1000 mg.

10. Pharmazeutische Zusammensetzung zur Verwendung bei der Behandlung von Infektionen oder Krankheiten, die durch gramnegative Bakterien der Ordnung Enterobacterales und/oder gramnegative Bakterien der Gattung Haemophilus verursacht oder verschlimmert werden, wobei die pharmazeutische Zusammensetzung die Verbindung (I) nach Anspruch 1 umfasst, oder ein pharmazeutisch annehmbares Salz, Hydrat oder Solvat davon und einen oder mehrere pharmazeutisch annehmbare Hilfsstoffe oder Träger, wobei die pharmazeutische Zusammensetzung bevorzugt durch intravenöse Infusion an einen Menschen verabreicht wird.

11. Pharmazeutische Zusammensetzung zur Verwendung bei der Behandlung von Infektionen oder Krankheiten, die durch gramnegative Bakterien der Ordnung Enterobacterales und/oder gramnegative Bakterien der Gattung Haemophilus verursacht oder verschlimmert werden, wobei die pharmazeutische Zusammensetzung die Verbindung (I) nach Anspruch 1 umfasst, oder ein pharmazeutisch annehmbares Salz, Hydrat oder Solvat davon und einen oder mehrere pharmazeutisch annehmbare Hilfsstoffe oder Träger, wobei die pharmazeutische Zusammensetzung durch intravenöse Infusion an einen Menschen verabreicht wird, mit der Verbindung (I) oder dem pharmazeutisch annehmbaren Salz, Hydrat oder Solvat davon, in einer Dosis von 50 bis 6000 mg pro Tag.

12. Pharmazeutische Zusammensetzung, zur Verwendung nach Anspruch 10, wobei die Verbindung (I) oder das pharmazeutisch annehmbare Salz, Hydrat oder Solvat davon einem Menschen verabreicht wird in einer Dosis von 50 bis 6000 mg pro Tag.

13. Pharmazeutische Zusammensetzung, zur Verwendung nach Anspruch 10, 11 oder 12, wobei die Verbindung (I) oder das pharmazeutisch annehmbare Salz, Hydrat oder Solvat davon einem Menschen verabreicht wird in einer Dosis von 50 bis 4000 mg pro Tag, z. B. 50 bis 3000 mg pro Tag, z. B. 50 bis 2000 mg pro Tag, z. B. 100 bis 2000 mg pro Tag, z. B. 200 bis 2000 mg pro Tag, z. B. 200 bis 1750 mg pro Tag, z. B. 200 bis 1500 mg pro Tag, z. B. 250 bis 1000 mg pro Tag.

14. Pharmazeutische Zusammensetzung, zur Verwendung nach einem der Ansprüche 11 bis 13, wobei die pharmazeutische Zusammensetzung einem Menschen durch intravenöse Infusion verabreicht wird, einmal täglich, zweimal täglich oder dreimal täglich, bevorzugt zweimal täglich; und/oder wobei die pharmazeutische Zusammensetzung zweimal täglich verabreicht wird, 1 bis 12 Stunden auseinander, z. B. 1 bis 8 Stunden auseinander oder 2 bis 6 Stunden auseinander oder 2 bis 5 Stunden auseinander, oder 3 bis 5 Stunden auseinander oder 1 bis 4 Stunden auseinander.

15. Pharmazeutische Zusammensetzung, zur Verwendung nach einem der Ansprüche 11 bis 14, wobei die pharmazeutische Zusammensetzung durch intravenöse Infusion an einen Menschen verabreicht wird und zur Verwendung bei der Behandlung von Atemwegsinfektionen mit gramnegativen Bakterien der Ordnung Enterobacterales und/oder gramnegativen Bakterien der Gattung Haemophilus bestimmt ist, und Harnwegsinfektionen (HWI) mit gramnegativen Bakterien der Ordnung Enterobacterales und/oder gramnegativen Bakterien der Gattung Haemophilus (typischerweise komplizierte HWI); und/oder wobei die pharmazeutische Zusammensetzung durch intravenöse Infusion einem Menschen verabreicht wird und die Behandlung von Infektionen oder Krankheiten, die durch gramnegative Bakterien der Ordnung Enterobacterales und/oder gramnegative Bakterien der Gattung Haemophilus verursacht oder verschlimmert werden, einen Behandlungsverlauf von 1 bis 10 Tagen bedingt, z. B. von 1 bis 7 Tagen oder 1 bis 5 Tagen.

## Revendications

1. Composé (I) : 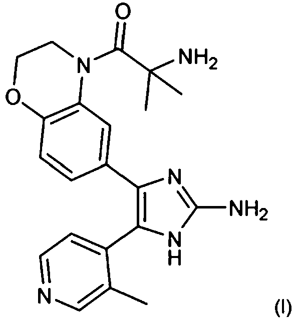 ou sel, hydrate ou solvate pharmaceutiquement acceptable de celui-ci.

2. Composé (I) selon la revendication 1, ou sel, hydrate ou solvate pharmaceutiquement acceptable de celui-ci, pour utilisation en thérapie ou en prophylaxie d'une infection par, ou d'une maladie causée ou exacerbée par, des bactéries Gram négatives de l'ordre des Enterobacterales et/ou des bactéries Gram négatives du genre Haemophilus.

3. Composé (I) selon la revendication 1, ou sel, hydrate ou solvate pharmaceutiquement acceptable de celui-ci, pour utilisation dans le traitement d'une infection par, ou d'une maladie causée ou exacerbée par, des bactéries Gram négatives de l'ordre des Enterobacterales et/ou des bactéries Gram négatives du genre Haemophilus.

4. Composé (I) pour utilisation selon la revendication 2 ou la revendication 3, ou sel, hydrate ou solvate pharmaceutiquement acceptable de celui-ci, dans lequel l'infection par, ou la maladie causée ou exacerbée par, des bactéries Gram négatives de l'ordre des Enterobacterales et/ou des bactéries Gram négatives du genre Haemophilus, est l'une ou plusieurs des suivantes : une bactériémie ou une infection sanguine par des bactéries Gram négatives de l'ordre des Enterobacterales et/ou des bactéries Gram négatives du genre Haemophilus, une infection respiratoire par des bactéries Gram négatives de l'ordre des Enterobacterales et/ou des bactéries Gram négatives du genre Haemophilus, une infection des voies urinaires (IVU) par des bactéries Gram négatives de l'ordre des Enterobacterales et/ou des bactéries Gram négatives du genre Haemophilus, une pyélonéphrite par des bactéries Gram négatives de l'ordre des Enterobacterales et/ou des bactéries Gram négatives du genre Haemophilus, et une infection intra-abdominale par des bactéries Gram négatives de l'ordre des Enterobacterales et/ou des bactéries Gram négatives du genre Haemophilus, de préférence, dans lequel l'infection par, ou la maladie causée ou exacerbée par, des bactéries Gram négatives de l'ordre des Enterobacterales et/ou des bactéries Gram négatives du genre Haemophilus, est l'une ou plusieurs des suivantes : une bactériémie ou une infection sanguine par des bactéries Gram négatives de l'ordre des Enterobacterales et/ou des bactéries Gram négatives du genre Haemophilus, une infection respiratoire par des bactéries Gram négatives de l'ordre des Enterobacterales et/ou des bactéries Gram négatives du genre Haemophilus, et une infection des voies urinaires (IVU) par des bactéries Gram négatives de l'ordre des Enterobacterales et/ou des bactéries Gram négatives du genre Haemophilus, et plus préférentiellement, dans lequel l'infection par, ou la maladie causée ou exacerbée par, des bactéries Gram négatives de l'ordre des Enterobacterales et/ou des bactéries Gram négatives du genre Haemophilus, est l'une ou plusieurs des suivantes : une infection respiratoire par des bactéries Gram négatives de l'ordre des Enterobacterales et/ou des bactéries Gram négatives du genre Haemophilus, et une infection des voies urinaires (IVU) par des bactéries Gram négatives de l'ordre des Enterobacterales et/ou des bactéries Gram négatives du genre Haemophilus ; et/ou dans lequel l'infection par, ou la maladie causée ou exacerbée par, des bactéries Gram négatives de l'ordre des Enterobacterales et/ou des bactéries Gram négatives du genre Haemophilus, est une infection touchant plusieurs sites.

5. Composé (I) pour utilisation selon la revendication 2, la revendication 3 ou la revendication 4, ou sel, hydrate ou solvate pharmaceutiquement acceptable de celui-ci, dans lequel le composé (I) ou un sel, un hydrate ou un solvate pharmaceutiquement acceptable de celui-ci est administré par voie parentérale, de préférence par voie intraveineuse, et plus préférentiellement, par perfusion intraveineuse (perfusion IV).

6. Composé (I) pour utilisation selon l'une quelconque des revendications 2 à 5, ou sel, hydrate ou solvate pharmaceutiquement acceptable de celui-ci, dans lequel les bactéries Gram négatives de l'ordre des Enterobacterales et/ou les bactéries Gram négatives du genre Haemophilus sont des Enterobacterales résistantes aux carbapénèmes ou des Enterobacterales productrices de β-lactamases à spectre élargi ; et/ou dans lequel les bactéries Gram négatives sont de l'ordre des Enterobacterales ; et/ou dans lequel les bactéries Gram négatives de l'ordre des Enterobacterales sont sélectionnées parmi les Enterobacterales des genres Arsenophonus, Atlantibacter, Biostraticola, Brenneria, Buchnera, Budvicia, Buttiauxella, Cedecea, Chania, Citrobacter, Cosenzaea, Cronobacter, Dickeya, Edwardsiella, Enterobacillus, Enterobacter, Erwinia, Escherichia, Ewingella, Franconibacter, Gibbsiella, Hafnia, Izhakiella, Kosakonia, Klebsiella, Kluyvera, Leclercia, Lelliottia, Leminorella, Levinea, Lonsdalea, Mangrovibacter, Moellerella, Morganella, Obesumbacterium, Pantoea, Pectobacterium, Phaseolibacter, Photorhabdus, Plesiomonas, Pluralibacter, Pragia, Proteus, Providencia, Pseudocitrobacter, Rahnella, Raoultella, Rosenbergiella, Rouxiella, Saccharobacter, Salmonella, Samsonia, Serratia, Shigella, Shimwellia, Siccibacter, Sodalis, Tatumella, Thorsellia, Trabulsiella, Wigglesworthia, Xenorhabdus, Yersinia et Yokenella, de préférence dans lequel les bactéries Gram négatives de l'ordre des Enterobacterales sont sélectionnées parmi Cedecea, Citrobacter, Erwinia, Escherichia, Enterobacter, Klebsiella, Kluyvera, Plesiomonas, Proteus, Providencia, Raoultella, Salmonella, Serratia, Shigella, et Yersinia, de préférence dans lequel les bactéries Gram négatives de l'ordre des Enterobacterales sont sélectionnées parmi Erwinia, Escherichia, Enterobacter, Klebsiella, Proteus, Salmonella, Serratia, Shigella, et Yersinia, de préférence dans lequel les bactéries Gram négatives de l'ordre des Enterobacterales sont sélectionnées parmi les Enterobacterales des genres Enterobacter, Escherichia et Klebsiella, et plus préférentiellement dans lequel les bactéries Gram négatives de l'ordre des Enterobacterales sont sélectionnées parmi les Enterobacterales des genres Escherichia et Klebsiella ; et/ou dans lequel les bactéries Gram négatives de l'ordre des Enterobacterales sont sélectionnées parmi Enterobacter spp., Escherichia coli et Klebsiella pneumoniae, de préférence Escherichia coli et Klebsiella pneumoniae.

7. Procédé d'inhibition in vitro de la croissance de bactéries Gram négatives de l'ordre des Enterobacterales et/ou de bactéries Gram négatives du genre Haemophilus en utilisant le composé (I) selon la revendication 1, ou un sel, un hydrate ou un solvate pharmaceutiquement acceptable de celui-ci.

8. Composé (I) : 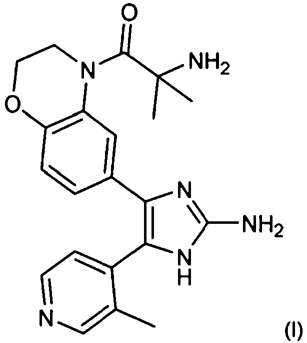 ou sel, hydrate ou solvate pharmaceutiquement acceptable de celui-ci, formulé avec un ou des excipient(s) ou véhicule(s) pharmaceutiquement acceptable(s) ; de préférence dans lequel le ou les excipient(s) ou véhicule(s) pharmaceutiquement acceptable(s) sont le ou les excipient(s) ou véhicule(s) pharmaceutiquement acceptable(s) approprié(s) pour une perfusion intraveineuse chez un sujet humain.

9. Composition pharmaceutique comprenant le composé (I) selon la revendication 1, ou un sel, un hydrate ou un solvate pharmaceutiquement acceptable de celui-ci, et un ou des excipient(s) ou véhicule(s) pharmaceutiquement acceptable(s) ; de préférence dans laquelle le ou les excipient(s) ou véhicule(s) pharmaceutiquement acceptable(s) sont un ou des excipient(s) ou véhicule(s) pharmaceutiquement acceptable(s) approprié(s) pour une perfusion intraveineuse chez un sujet humain ; et/ou dans laquelle le composé (I), ou un sel, un hydrate ou un solvate pharmaceutiquement acceptable de celui-ci, est présent en une quantité de 50 à 6 000 mg, de préférence 50 à 4 000 mg, telle que 50 à 3 000 mg, telle que 50 à 2 000 mg, telle que 100 à 2 000 mg, telle que 200 à 2 000 mg, telle que 200 à 1 750 mg, telle que 200 à 1 500 mg, telle que 250 à 1 000 mg.

10. Composition pharmaceutique pour utilisation dans le traitement d'une infection par, ou d'une maladie causée ou exacerbée par, des bactéries Gram négatives de l'ordre des Enterobacterales et/ou des bactéries Gram négatives du genre Haemophilus, la composition pharmaceutique comprenant le composé (I) selon la revendication 1, ou un sel, un hydrate ou un solvate pharmaceutiquement acceptable de celui-ci, et un ou des excipient(s) ou véhicule(s) pharmaceutiquement acceptable(s), de préférence dans laquelle la composition pharmaceutique est administrée par perfusion intraveineuse à un sujet humain.

11. Composition pharmaceutique pour utilisation dans le traitement d'une infection par, ou d'une maladie causée ou exacerbée par, des bactéries Gram négatives de l'ordre des Enterobacterales et/ou des bactéries Gram négatives du genre Haemophilus, la composition pharmaceutique comprenant le composé (I) selon la revendication 1, ou un sel, un hydrate ou un solvate pharmaceutiquement acceptable de celui-ci, et un ou des excipient(s) ou véhicule(s) pharmaceutiquement acceptable(s), dans laquelle la composition pharmaceutique est administrée par perfusion intraveineuse à un sujet humain, avec le composé (I), ou un sel, un hydrate ou un solvate pharmaceutiquement acceptable de celui-ci, à la posologie de 50 à 6 000 mg par jour.

12. Composition pharmaceutique pour utilisation selon la revendication 10, dans laquelle le composé (I), ou un sel, un hydrate ou un solvate pharmaceutiquement acceptable de celui-ci, est administré à un sujet humain à la posologie de 50 à 6 000 mg par jour.

13. Composition pharmaceutique pour utilisation selon la revendication 10, la revendication 11 ou la revendication 12, dans laquelle le composé (I), ou sel, hydrate ou solvate pharmaceutiquement acceptable de celui-ci, est administré à un sujet humain à la posologie de 50 à 4 000 mg par jour, telle que 50 à 3 000 mg par jour, telle que 50 à 2 000 mg par jour, telle que 100 à 2 000 mg par jour, telle que 200 à 2 000 mg par jour, telle que 200 à 1 750 mg par jour, telle que 200 à 1 500 mg par jour, telle que 250 à 1 000 mg par jour.

14. Composition pharmaceutique pour utilisation selon l'une quelconque des revendications 11 à 13, dans laquelle la composition pharmaceutique est administrée par perfusion intraveineuse à un sujet humain une fois par jour, deux fois par jour ou trois fois par jour, de préférence deux fois par jour ; et/ou dans laquelle, lorsque la composition pharmaceutique est administrée deux fois par jour, les deux administrations sont espacées de 1 à 12 heures, tel que de 1 à 8 heures, ou de 2 à 6 heures, ou de 2 à 5 heures, ou de 3 à 5 heures, ou de 1 à 4 heures.

15. Composition pharmaceutique pour utilisation selon l'une quelconque des revendications 11 à 14, dans laquelle la composition pharmaceutique est administrée par perfusion intraveineuse à un sujet humain, et est à utiliser dans le traitement d'infections respiratoires par des bactéries Gram négatives de l'ordre des Enterobacterales et/ou des bactéries Gram négatives du genre Haemophilus, et d'infections des voies urinaires (IVU) par des bactéries Gram négatives de l'ordre des Enterobacterales et/ou des bactéries Gram négatives du genre Haemophilus (généralement des IVU compliquées) ; et/ou dans laquelle la composition pharmaceutique est administrée par perfusion intraveineuse à un sujet humain et le traitement d'une infection par, ou d'une maladie causée ou exacerbée par, des bactéries Gram négatives de l'ordre des Enterobacterales et/ou des bactéries Gram négatives du genre Haemophilus implique une série de traitement de 1 à 10 jours, tel que de 1 à 7 jours, ou 1 à 5 jours.
